(19) Europäisches Patentamt
European Patent Office
Office européen des brevets

(11) Veröffentlichungsnummer: **0 392 389 A2**

(12) **EUROPÄISCHE PATENTANMELDUNG**

(21) Anmeldenummer: **90106640.7**

(22) Anmeldetag: **06.04.90**

(51) Int. Cl.5: **C07D 311/72, C07C 69/96, C07C 69/347**

(30) Priorität: **14.04.89 CH 1411/89**
**30.01.90 CH 288/90**

(43) Veröffentlichungstag der Anmeldung:
**17.10.90 Patentblatt 90/42**

(84) Benannte Vertragsstaaten:
**AT BE CH DE DK FR GB IT LI NL**

(71) Anmelder: **F. HOFFMANN-LA ROCHE AG**
**Postfach 3255**
**CH-4002 Basel(CH)**

(72) Erfinder: **Knierzinger, Andreas, Dr.**
**Burenweg 30**
**CH-4127 Birsfelden(CH)**
Erfinder: **Scalone, Michelangelo, Dr.**
**Baslerstrasse 14**
**CH-4127 Birsfelden(CH)**

(74) Vertreter: **Urech, Peter, Dr. et al**
**Grenzacherstrasse 124 Postfach 3255**
**CH-4002 Basel(CH)**

(54) **Verfahren zur Herstellung von Vinylverbindungen.**

(57) Die Erfindung betrifft ein Verfahren zur Herstellung von Vinylchromanen. Dieses Verfahren beinhaltet die Herstellung der Verbindungen der Formel

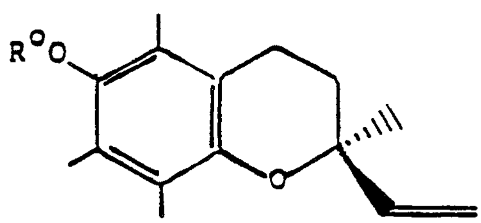

I

worin R° Wasserstoff oder eine abspaltbare Schutzgruppe ist,
und ist dadurch gekennzeichnet, dass man eine Verbindung der Formel

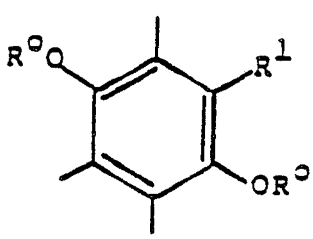

II

worin R° obige Bedeutung besitzt und $R^1$ den Rest $-CH_2-CH_2-C(W)(CH_3)-CH=CH_2$ oder $CH_2CH_2-C(CH_3)-=CH-CH_2-W$ darstellt, worin W eine Abgangsgruppe ist,
mittels eines chiralen Uebergangsmetall-diphosphinkomplexes cyclisiert.

Die Verbindungen I sind wertvolle Zwischenprodukte, beispielsweise geeignet zur Herstellung von (R,R,R)-α-Tocopherol.

EP 0 392 389 A2

## Verfahren zur Herstellung von Vinylverbindungen

Die Erfindung betrifft ein Verfahren zur Herstellung von Vinylchromanen. Dieses Verfahren beinhaltet die Herstellung der Verbindungen der Formel

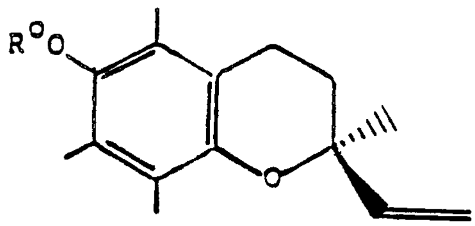

I

worin $R^{o}$ Wasserstoff oder eine abspaltbare Schutzgruppe ist,
und ist dadurch gekennzeichnet, dass man eine Verbindung der Formel

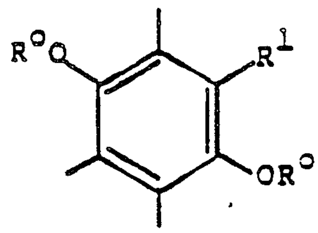

II

worin $R^{o}$ obige Bedeutung besitzt und $R^1$ den Rest $-CH_2-CH_2-C(W)(CH_3)-CH=CH_2$ oder $-CH_2-CH_2-C(CH_3)=CH-CH_2-W$ darstellt, worin W eine Abgangsgruppe ist,
mittels eines chiralen Uebergangsmetall-diphosphinkomplexes cyclisiert.

Die zwei Substituenten $R^{o}$ können gleich oder voneinander verschieden sein.

W charakterisiert zweckmässigerweise eine Estergruppierung, beispielweise der Formeln

$-O\underset{\underset{O}{\|}}{C}R'$

$-O\underset{\underset{O}{\|}}{C}OR''$

$-O\underset{\underset{O}{\|}}{P}(OR'')_2$

worin bedeuten:
$R'$ = nieder-Alkyl, Perfluor-$C_{1-20}$-alkyl, Aryl
$R''$ = nieder-Alkyl, Aryl, Benzyl.

Durch W kann anderseits auch ein Halogenid oder eine Aryloxygruppe charakterisiert werden.

Der Ausdruck "nieder-Alkyl" bedeutet im Rahmen der vorliegenden Erfindung geradkettige oder verzweigte Alkylgruppen mit 1 bis 9 Kohlenstoffatomen wie z.B. Methyl, Ethyl, Propyl, Isopropyl, n-Butyl, Isobutyl, tert. Butyl, Pentyl, Hexyl, Heptyl, Octyl oder Nonyl und dergleichen.

Der Ausdruck "Perfluor-$C_{1-20}$-alkyl" bedeutet im Rahmen der vorliegenden Erfindung sowohl geradkettige, als auch verzweigte, gegebenenfalls auch optisch aktive Ketten, wobei nicht unbedingt sämtliche Wasserstoffatome durch Fluoratome ersetzt sein müssen. Falls nicht alle Wasserstoffatome durch Fluoratome ersetzt sind, so ist häufig insbesondere noch ein endständiges Wasserstoffatom vorhanden.

Der im Zusammenhang mit den Verbindungen der Formeln III und IV verwendete Ausdruck "Aryl" bedeutet im Rahmen der vorliegenden Erfindung sowohl aromatische Kohlenwasserstoffe als auch aromatische Heterocyclen, insbesondere solche mit 4 bis 14 Kohlenstoffatomen. Als Heteroatome kommen insbesondere Sauerstoff und Stickstoff in Betracht. Weiterhin können die Ringe sowohl unsubstituiert als auch substituiert sein, wobei als Substituent bevorzugt in Betracht kommen Halogen, Hydroxy, nieder-Alkyl, Perfluor-$C_{1-20}$-alkyl, niederes Alkoxy und Formyl. Eine vorhandene Arylgruppe kann zudem komplex an ein Uebergangsmetall wie Chrom, Eisen oder auch Nickel gebunden sein.

Der im nachfolgenden im Zusammenhang mit den Verbindungen der Formel II, IX und X verwendete

Ausdruck "Aryl" bedeutet im Rahmen der vorliegenden Erfindung Phenyl, welches gegebenenfalls in ortho-, meta- oder para-Stellung nieder-Alkyl oder nieder-Alkoxygruppen, oder auch di-nieder-Alkylamino, vorzugsweise Dimethylaminogruppen, aufweisen kann.

Der Ausdruck "Halogenid" bedeutet im Rahmen der vorliegenden Erfindung Fluorid, Chlorid, Bromid und Jodid, wobei Chlorid bevorzugt ist. Der Ausdruck "Halogen" bedeutet im Rahmen der vorliegenden Erfindung Fluor, Chlor, Brom und Jod, wobei Fluor bevorzugt ist. Der Ausdruck "Aryloxy" bedeutet Gruppen, in denen der Arylrest die vorhergehende Bedeutung hat. Dasselbe gilt für Aroyl. Der Ausdruck "Benzyl" bedeutet $CH_2$-Aryl, wobei der Arylrest die vorhergehende Bedeutung hat. Der Ausdruck "nieder-Alkoxy" steht für Gruppen, in denen der Alkylrest die vorhergehende Bedeutung hat. Dasselbe gilt für "nieder-Alkanoyl". Weiterhin bedeutet das Zeichen

" 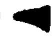 ",

dass sich der entsprechende Rest oberhalb der Molekulebene befindet, während das Zeichen

" 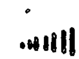 "

bedeutet, dass sich der entsprechende Rest unterhalb der Molekülebene befindet.

Als Uebergangsmetall fungiert insbesondere ein Metall der Gruppe 8 des periodischen Systems, z.B. Rhodium, Palladium, Nickel oder Platin, insbesondere aber Rhodium und Palladium.

Beispiele von geeigneten Komplexen sind die Rh-Komplexe der europäischen Patentpublikationen Nr. 158 875 und Nr. 218 970 [solche Komplexe werden hier für asymmetrische Hydrierungen verwendet], also die Verbindungen der Formel

$[Rh(X)(Y)(L_{0,1,2})]_{1,2}$ III

In der Formel III bedeuten demnach :

X : Halogenid, $BF_4^-$ , $PF_6^-$ , $ClO_4^-$ , $NO_3^-$ , $B(C_6H_5)_4^-$ , oder ein Rest der Formel Z-$COO^-$ , gegebenenfalls auf einem Träger fixiert

Z : 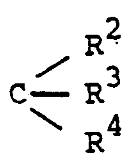 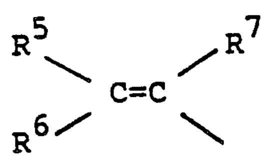 ; Aryl

worin sind: $R^2$, $R^3$, $R^4$: H; Halogen; nieder Alkyl; Aryl-nieder-alkyl; Perfluor-$C_{1-20}$-alkyl; Aryl; $OR^8$ ; $-(CH_2)_n$-COA ; AOC-$(CF_2)_n$;

wenigstens einer von $R^2$, $R^3$ und $R^4$ ist $OR^8$, Aryl oder Halogen.

$R^5$, $R^6$, $R^7$ : H; Halogen; nieder Alkyl; Aryl-nieder alkyl; Perfluor-$C_{1-20}$-Alkyl; Aryl; $(CH_2)_n$-COA ; AOC-$(CF_2)_n$.

$R^8$ : H; nieder Alkyl; teilweise oder vollständig halogeniertes niederes Alkyl; Aryl; Aryl-nieder-alkyl.

A : $-OR'''$; $NR''''_2$. Die Substituenten $R''''$ können gleich oder verschieden sein.

$R'''$ : H; nieder Alkyl; Aryl; Aryl-nieder-alkyl; Kation.

$R''''$: H; nieder Alkyl; Aryl; Aryl-nieder-alkyl.

n : Zahl 0-20, insbesondere 0-5

Y : chiraler Diphosphinligand

L : neutraler Ligand

Der Ausdruck "neutraler Ligand" bedeutet im Rahmen der vorliegenden Erfindung einen leicht austauschbaren Liganden. Beispiele sind: Olefine, z.B. Aethylen, Propylen, Cycloocten, 1,5-Hexadien, Norbornadien, 1,5-Cyclooctadien und dergleichen, Nitrile wie Acetonitril, Benzonitril, oder auch das verwendete Lösungsmittel usw. Ein Olefinligand kann gegebenenfalls durch vorherige Hydrierung entfernt werden. Falls mehr als ein solcher Ligand vorhanden ist, können diese auch voneinander verschieden sein.

Als chirale Diphosphinliganden können im Prinzip beliebige, im Zusammenhang mit asymmetrischen Hydrierungen bekannte Diphosphine, welche gegebenenfalls auch auf einem Träger fixiert sein können, verwendet werden. Derartige Liganden sind bekannt und dem Fachmann leicht zugänglich. Beispielsweise sind im Rahmen der vorliegenden Erfindung im Frage kommende Liganden bekannt aus Literaturstellen, die sich mit Hydrierungen befassen: Marko, L. et al. Aspects of Homogeneous Catalysis, 4, 145-202 (1981);

Europäischen Patentpublikation Nr. 245 959; Schmid R. et al., Helv. Chim. Acta, 71, 897-929 (1988). Besonders geeignete Liganden sind z.B. die chiralen Diphosphine der allgemeinen Formeln

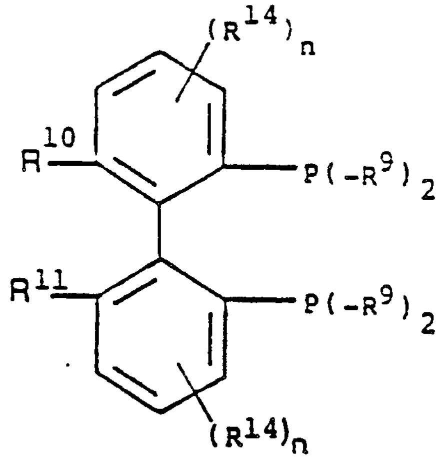

IX

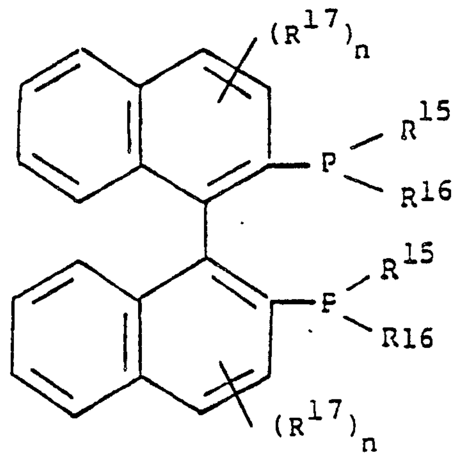

X

Bevorzugte Diphosphinliganden sind solche der Formel IX und X, worin
$R^9$ : Aryl, Cyclohexyl
$R^{10}$, $R^{11}$: H; nieder-Alkyl; nieder-Alkoxy; di-nieder-Alkylamino; geschütztes Hydroxymethyl. $R^{10}$ und $R^{11}$ können gegebenenfalls voneinander verschieden sein oder zusammen die folgenden Gruppen bedeuten:

$(-CH_2)_m$; $-CH_2-O-CH_2-$;

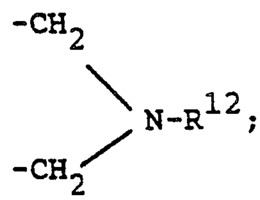

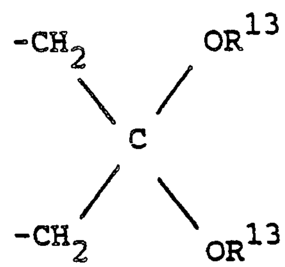

m : Zahl 3,4,5
$R^{12}$ : nieder Alkyl, Aryl, Benzyl
$R^{13}$ : nieder Alkyl; oder beide $R^{13}$ zusammen Di- oder Trimethylen
$R^{14}$ : $-CH_3$; nieder Alkoxy; di-nieder Alkylamino; Halogen,
n : Zahl 0,1,2,3
$R^{15}$, $R^{16}$: Aryl, Cyclohexyl
$R^{17}$ : Methyl, Ethyl, Halogen, -OH; $NH_2$, Acetylamino, Nitro, $-SO_3H$, bevorzugt in der 5,5′-Position bedeuten.

Als Beispiele von besonders bevorzugten Diphosphinliganden können folgende genannt werden:
(6,6′-Dimethylbiphenyl-2,2′-diyl)bis(diphenylphosphin) [BIPHEMP];
6,6′-(Dimethylbiphenyl-2,2′-diyl)-bis(di-m-tolylphosphin) [mTolBIPHEMP]
(6,6′-Dimethylbiphenyl-2,2′-diyl)bis(di-p-tolylphosphin) [pTolBIPHEMP];
2,2′-Bis(diphenylphosphino)-1,1′-binaphthyl [BINAP];
2,2′-Bis(di-p-tolylphosphino)-1,1′-binaphthyl [pTolBINAP].
2,2′-Bis(di-m-tolylphosphino)-1,1′-binaphtyl [mTolBINAP]
6,6′-Dimethoxybiphenyl-2,2′-diyl)-bis(diphenylphosphin) [MeOBIPHEMP]

Bevorzugte Rhodium-diphosphin-Komplexe der Formel III sind solche, worin Z die Gruppe

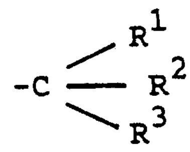

darstellt, wobei einer der Substituenten $R^1$, $R^2$ und $R^3$ Fluor, und die beiden anderen Halogen, Perfluor-$C_{1-20}$ -alkyl oder Aryl darstellen. Falls der Ligand X chiral ist, kann dieser in racemischer [(R):(S) = 1:1] oder, bevorzugt, in optisch aktiver Form vorliegen.

Die Rh-Komplexe der Formel III können gemäss europäischer Patentpublikation Nr. 158 875 oder z.B. aus der Reaktion von [(1,5-Cyclooctadien)($X^1$)Rh] mit einem Diphosphinliganden Y (gemäss Fryzuk M.D., Inorg.Chem., 21, 2134-2139 (1982) [wissenschaftliche Arbeit; ohne Verwendungsangaben für die Produkte]) und einer Säure der Formel Z-COOH hergestellt werden, wobei $X^1$ ein $\pi$-allylischer Ligand vom Typ

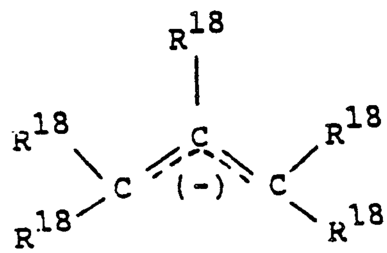

darstellt, worin $R^{18}$ Wasserstoff, Aryl, nieder Alkyl oder Benzyl bedeutet, und Y bzw. Z obige Bedeutung haben. Die Substituenten $R^{18}$ können auch voneinander verschieden sein.

Die Konfiguration des Liganden Y bestimmt die Konfiguration des Produktes I. Um zu dem erfindungsgemässen (S)-Vinylchroman I (Vorläufer für (R,R,R)-$\alpha$-Tocopherol) zu gelangen, wird der Ligand Y je nach Art des Katalysators und der Reaktionsbedingungen in der (R)- oder (S)-Form eingesetzt: eine Bestimmung der Konfiguration des Materials I ergibt hierfür die nötige Auskunft auf einfache Weise.

Als geeignet haben sich ferner Komplexe der Formel

[Pd(X)($X^1$)(Y)] IV

erwiesen, worin X, $X^1$ und Y obige Bedeutung haben.

Diese letzteren Komplexe sind in an sich bekannter, einfacher Weise, z.B. B.Bosnich et al., J.Am.Chem.Soc.,107, 2033-2045 (1985) "Asymmetric Synthesis. Asymmetric Catalytic Allylation Using Palladium Chiral Phosphine Complexes", aus dem entsprechenden Diphosphin, einer Verbindung [Pd(X)-($X^1$)$]_{1,2}$ und gegebenenfalls einem Salz mit dem Säurerest X zugänglich.

Die Verbindungen III und IV können für sich synthetisiert oder in situ in Gegenwart der zu cyclisierenden Verbindung II aus den Komponenten hergestellt werden.

Die erfindungsgemässe Cyclisierung wird zweckmässigerweise in einem Temperaturbereich von ca. -20°C bis ca. 100°C, insbesondere in einem Bereich von ca. 0°C und 60°C durchgeführt.

Man arbeitet zweckmässigerweise in einem Lösungsmittel, insbesondere einem gegebenenfalls halogenierten aliphatischen oder aromatischen Kohlenwasserstoff, z.B. Methylenchlorid, Chloroform oder Toluol, einem Ether, z.B. Tetrahydrofuran oder Dioxan, einem Ester, z.B. Ethylacetat, einem Alkohol, z.B. Methanol, Ethanol, tert-Butanol, einem Amid, z.B. Dimethylformamid, einem Sulfoxid, z.B. Dimethylsulfoxid, in einer oder in Anwesenheit einer organischen Base, z.B. Pyridin, Dimethylaminopyridin. In gewissen Fällen ist es vorteilhaft, die Verbindungen II durch Reaktion mit einer anorganischen Base, z.B. Natriumhydrid, Calciumhydrid, Lithiumhydrid, Kaliumhydrid, vor der Cyclisierung in ein Salz umzuwandeln.

Die Menge des Katalysators ist nicht kritisch. Der Katalysator wird aber zweckmässigerweise in einer Menge von 0.01-10 Gew.% (bezogen auf die Verbindung II), vorzugsweise 1-5 Gew.%, eingesetzt.

Die Schutzgruppe $R^{\circ}$, also insbesondere eine Gruppe ausgewählt aus: nieder-Alkyl, Alkanoyl, Aroyl oder gegebenenfalls substituiertes Benzyl wird nach der erfindungsgemässen Cyclisierung entweder sauer, basisch oder durch Hydrogenolyse in an sich bekannter Weise abgespalten.

Die gegenständliche Synthese erlaubt es, optisch aktive Chromanringbausteine für die Herstellung von (R,R,R)-$\alpha$-Tocopherol in guter chemischer und optischer Ausbeute ("enantiomeric excess") zu gewinnen, Zwischenprodukte, die zudem am $C_2$-Atom bereits die Konfiguration des (R,R,R)-$\alpha$-Tocopherols aufweisen.

Eine erfindungsgemässe erhaltene Verbindung I wird zweckmässigerweise durch Hydroborierung und Oxidation in eine Verbindung der Formel

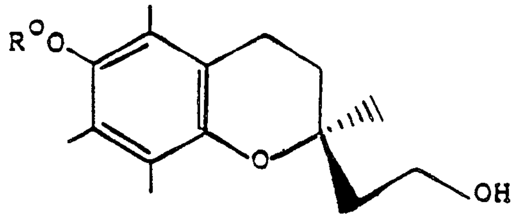

VI

worin $R^{\circ}$ Wasserstoff oder eine abspaltbare Schutzgruppe darstellt,
übergeführt, z.B. gemäss H.C. Brown, J.C. Chen, J.Org. Chem., 46, 3988 (1981), wo als Hydroborierungsmittel 3-Borabicyclo-[3,3,1]nonan und als Oxidationsmittel Wasserstoffperoxid eingesetzt werden.

Die Verbindung VI kann hierauf in an sich bekannter Weise in (R,R,R)-α-Tocopherol übergeführt werden, siehe z.B. N. Cohen et al., J. Org. Chem., 41, 3505 (1976), wo die Aktivierung des funktionellen Rests der Verbindung VI und die Kopplung mit dem erforderlichen $C_{14}$-Seitenkettenderivat beschrieben ist.

Die Verbindungen der Formel II können nach an sich bekannter Methodik hergestellt werden.

Die racemischen Verbindungen

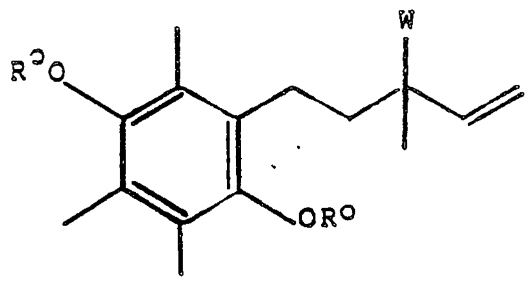

IIa

sind z.B. zugänglich aus den entsprechend substituierten Chromanen der Formel V

V $\rightleftharpoons$ Va

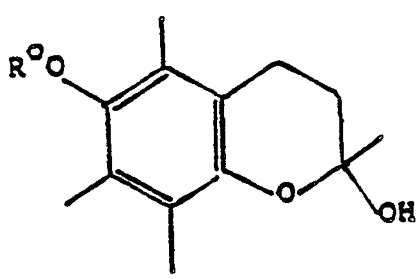

V

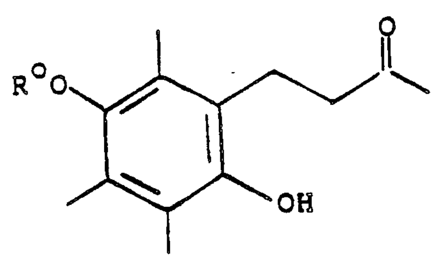

Va

Das Halbacetal V steht je nach Bedingungen im Gleichgewicht mit dem Keton Va. Die Verbindungen V sind bekannt, siehe z.B. N. Cohen et al., Helv.Chim.Acta, 61, 837-843 (1978), oder können in Analogie zu dieser bekannten Methode hergestellt werden.

V (bzw. Va) kann nach an und für sich bekannten Methoden, z.B. durch Umsetzung mit einer Vinylgrignard-Verbindung und anschliessender Veresterung in IIa übergeführt werden, wobei es meistens vorteilhaft ist, die reaktivere phenolische OH-Gruppe zuerst auf übliche Weise, z.B. durch Silylierung, zu schützen.

Prochirale Verbindungen der Formel IIb und IIc, worin $R^{\circ}$ und W obige Bedeutung haben, können ebenfalls ausgehend von V hergestellt werden, z.B. via Allylumlagerung von IIa, in Analogie zu L.E.

Overman und F.M. Knoll, Tetrahedron Letters (1979), 321 seq.: "Palladium (II) - Catalyzed Rearrangement of Allylic Acetates".

Die Verbindungen der Formel IIb und IIc sind anderseits auch aus geeignet substituierten Trimethylhydrochinonderivaten erhältlich, indem man hier einen geeigneten, reaktiven Substituenten entsprechend zum Rest R[1] verlängert.

Solche reaktive Trimethylhydrochinonderivate sind beispielweise

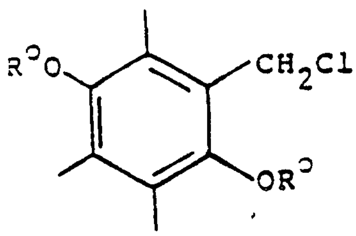

VII

bzw. das daraus durch Kopplung mit einer Grignardverbindung erhältliche

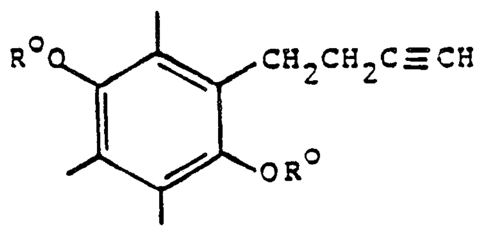

VIII

wobei R° obige Bedeutung hat. Beide R° können gleich oder verschieden sein.

Die weitere Umsetzung von VIII kann nach an sich dem Fachmann bekannten Methoden durchgeführt werden, wozu die üblichen selektiven Methoden zur Herstellung von trisubstituierten Doppelbindungen in der (E)- bzw. (Z)-Form dienen können.

Die an beiden phenolischen OH-Gruppen geschützte Verbindung IIb (W=OH), wo die Doppelbindung in der (E)-Form vorliegt, kann z.B. durch Carbometallierung und Hydroxymethylierung der Dreifachbindung in Analogie zu E.I. Negishi et al., J.Org.Chem., 45, 2526 (1980) "Conversion of Methyl Ketones into Terminal Acetylenes and (E)-Trisubstituted Olefins of Terpenoid Origin", hergestellt werden. Die am beiden phenolischen OH-Gruppen geschützte Verbindung IIc (W=OH), wo die Doppelbindung in der (Z)-Form vorliegt, kann hingegen durch Methoxycarbonylierung, nucleophile Methylierung der Dreifachbindung mit Kupferverbindungen in Analogie zu G.H. Posner, Organic Reactions, 19, 45/46 (1972) "α,β-Acetylenic Carbonyl Derivatives" (stereoselektive Synthese) und nachfolgender Reduktion der Estergruppe erhalten werden.

Die anschliessende Veresterung der allylischen Hydroxygruppe gefolgt von der oxidativen Spaltung der Schutzgruppen und Reduktion der Chinone zu den Hydrochinonen ergibt nach der dem Fachmann bestbekannter Methodik die bzgl. W und R° geeignetsubstituierten Trimethylhydrochinonderivate IIb und IIc in reiner (E)-bzw. (Z)-Form.

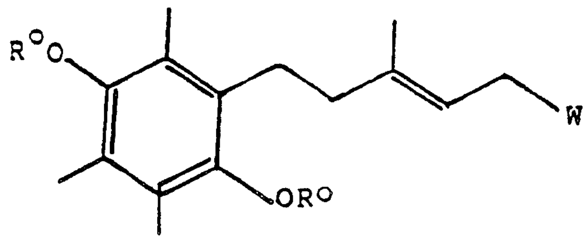

IIb

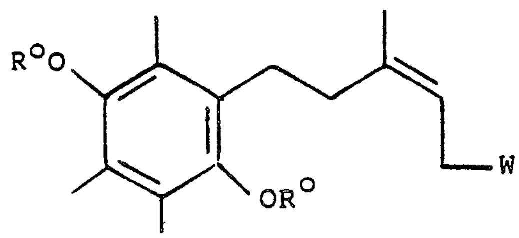

IIc

## Beispiele

Die folgenden Beispiele dienen zur Erläuterung der Erfindung und stellen keinerlei Einschränkung hiervon dar. In diesen Beispielen haben die verwendeten Abkürzungen folgende Bedeutung:
COD = 1,5-Cyclooctadien
BINAP = 2,2′Bis(diphenylphosphino)-1,1′-binaphthyl
BIPHEMP = 6,6′-(Dimethylbiphenyl-2,2′-diyl)bis(diphenylphosphin)
mTolBIPHEMP = 6,6′-(Dimethylbiphenyl-2,2′-diyl)-bis(di-m-tolylphosphin)
pTolBIPHEMP = 6,6′-(Dimethylbiphenyl-2,2′-diyl)-bis(di-p-tolylphosphin)
mTolBINAP = 2,2′-Bis(di-m-tolylphosphino)-1,1′-binaphtyl
pTolBINAP = 2,2′-Bis(di-p-tolylphosphino)-1,1′binaphtyl
MeOBIPHEP = 6,6′-(Dimethoxybiphenyl-2,2′-diyl)-bis(diphenylphosphin)
9-BBN = 9-Borabicyclo-[3.3.1]nonan

Die enantiomere Zusammensetzung (optische Reinheit) der Cyclisierungsprodukte basiert auf der spezifischen optischen Drehung (589 nm, 20°) der reinen Vinylchromane der Formel I mit (S)-Konfiguration:
R° = H , [α] = -89.0° (c = 0.5 , Chloroform)
R° = Methyl, [α] = -77.5° (c = 0.3 , Chloroform)
R° = Acetyl, [α] = -79.5° (c = 1.0 , Octan) 1]
R° = Benzyl, [α] = -58.7° (c = 1.0 , Chloroform)

A₁ Racemische DIOLE Typ IIa

Beispiel 1

Eine Lösung von 10.0 g rac. 6-Benzyloxy-2-hydroxy-2,5,7,8-tetramethylchroman (32.0 mMol) in THF (50

1] H.J. Mayer. P. Schudel. R. Rüegg und O. Isler. Helv. Chim. Acta, 46 , 963 (1963); das Verfahren ist kommerziell unattraktiv; der Chromanbaustein wird nach einem nicht - katalytischen Verfahren hergestellt; Racematspaltung zeitigt also im Minimum 50 % Verlust; opt. aktives Spaltungsreagens ist teuer, muss deshalb recycliert werden.

ml) wird bei -20°C tropfenweise mit 68.4 ml einer Lösung von 1.3M Vinylmagnesium-bromid (88.4 mMol) versetzt. Nach beendeter Zugabe wird das Reaktionsgemisch auf Raumtemperatur erwärmt und 2 weitere Stunden gerührt.

Danach wird das Reaktionsgemisch bei 0°C mit einer gesättigten Ammoniumchloridlösung (70 ml) gewaschen. Der weisse Niederschlag (anorganische Salze) wird abgenutscht, mit Ether gewaschen und verworfen. Die wässrige Phase wird mit Ether extrahiert, die vereinigten organischen Phasen werden getrocknet und eingedampft. Die Kristallisation des öligen Rückstandes aus Ether/Hexan ergibt 9.30 g 2-[-(R,S)-3-Hydroxy-3-methyl-4-pentenyl]-3,5,6-trimethyl-4-benzyloxyphenol mit einem Schmelzpunkt von 96-99°C.

Analog werden weitere Diole hergestellt.

Aus rac. 6-Acetoxy-2-hydroxy-2,5,7,8-tetramethylchroman : 4-Hydroxy-3-(3-hydroxy-3-methyl-4-pentenyl)-2,5,6-trimethylphenyl acetat (Smp. 104-105 °C).

Aus rac. 6-Methoxy-2-hydroxy-2,5,7,8-tetramethylchroman : 2-[(R,S)-3-Hydroxy-3-methyl-4-pentenyl]-4-methoxy-3,5,6-trimethylphenol (farbloses Oel; MS: 264(M+), 246(-$H_2O$), 231(-$H_2O$,-$CH_3$), 217, 179, 178; IR: 3387, 2932, 2829, 1454, 1409, 1251, 1084).

$A_2$ Racemische ESTER

Beispiel 2

Eine Lösung von 10.0 g 2-[(R,S)-3-Hydroxy-3-methyl-4-pentenyl]-3,5,6-trimethyl-4-benzyloxyphenol (29.4mMol) in THF (75 ml) wird bei 0°C tropfenweise mit 39.5 ml einer Lösung von 1.6M Butyllithium in Hexan (63.3 mMol) versetzt und 1 Stunde gerührt. Danach werden zuerst eine Lösung von 5.0 ml Trimethylchlorsilan (39.1 mMol) in THF (8 ml) und nach weiteren 1.5 Stunden eine Lösung von 3.0 ml Acetanhydrid (31.7 mMol) in THF (7.5 ml) unter Rühren bei 0°C zugetropft. Nach 2 Stunden wird das Reaktionsgemisch bei 0°C mit einer gesättigten Ammoniumchloridlösung (50 ml) gewaschen, die wässrige Phase mit Ether extrahiert und verworfen. Die vereinigten organischen Phasen werden getrocknet und das nach Entfernen des Lösungsmittels verbleibende Oel wird an Kieselgel (Laufmittel: Ether/Hexan 1:3) gereinigt. Eine Lösung des 8.4 g isolierten, farblosen Oels (18.5 mmol) in THF (52 ml) wird bei 0°C mit einer Lösung von Tetrabutylammoniumfluorid (6.2 g, 19.7 mmol) in THF (21 ml) versetzt. Nach 1 Stunde Rühren bei 0°C wird das Reaktionsgemisch mit einer gesättigten Ammoniumchloridlösung (35 ml) gewaschen. Die wässrige Phase wird mit Ether extrahiert, die vereinigten organischen Phasen werden getrocknet und eingedampft. Nach Filtration des Rückstandes auf Kieselgel ergibt die Kristallisation aus Ether/ n-Hexan 5.86 g (R,S)-1-[3-Benzyloxy-6-hydroxy-2,4,5-trimethylphenethyl]-1-methylallyl acetat mit einem Schmelzpunkt von 62-63.5°C.

Analog werden weitere Ester hergestellt.

Aus 2-[(R,S)-3-Hydroxy-3-methyl-4-pentenyl]-3,5,6-trimethyl-4-benzyloxyphenol:

(R,S)-1-[3-Benzyloxy-6-hydroxy-2,4,5-trimethylphenetyl]-1-methylallyl butyrat (Smp. 78-79°C).

(R,S)-1-[3-Benzyloxy-6-hydroxy-2,4,5-trimethylphenethyl]-1-methylallyl methylcarbonat (Smp. 59.5-61 °C).

(R,S)-1-[3-Benzyloxy-6-hydroxy-2,4,5-trimethylphenethyl]-1-methylallyl tert-butylcarbonat (Smp. 72-73°C).

Aus 2-[(R,S)-3-Hydroxy-3-methyl-4-pentenyl]-4-methoxy-3,5,6-trimethylphenol:

rac-1-(2-Hydroxy-5-methoxy-3,4,6-trimethylphenethyl)-1-methylallyl acetat (farbloses Oel; MS: 306 (M+), 246 (-AcOH) 231, 179, 178; IR: 3474, 1736, 1252).

rac-1-(2-Hydroxy-5-methoxy-3,4,6-trimethylphenethyl)-1-methylallyl methylcarbonat (Smp. 62.5-63°C).

rac-1-(2-Hydroxy-5-methoxy-3,4,6-trimethylphenethyl)-1-methylallyl benzylcarbonat (farbloses Oel; MS: 398 (M+), 246 (-BzOCOOH), 178; IR: 3512, 1743, 1265).

rac-1-(2-Hydroxy-5-methoxy-3,4,6-trimethylphenethyl)-1-methylallyl tert-butylcarbonat (farbloses Oel; MS: 364 (M+), 246 (- t-BuOCOOH), 57; IR: 3508, 1739, 1285, 1252).

Aus 4-Hydroxy-3-(3-hydroxy-3-methyl-4-pentenyl)- 2,5,6-trimethylphenyl acetat:

rac-1-(3-Acetoxy-6-hydroxy-2,4,5-trimethylphenethyl)-1-methylallyl methylcarbonat, Smp. 114-116°C.

B. CYCLISIERUNGEN

Beispiel 3

a) SUBSTRAT Typ IIa : (R,S)-1-[3-Benzyloxy-6-hydroxy-2,4,5-trimethylphenethyl]-1-methylallyl acetat

Versuch 1

In einer Dry-Box ($O_2$-Gehalt < 1ppm) werden in einem 25 ml Schlenkrohr 6.8 mg $[Rh(CF_3COO)(COD)]_2$ (0.0105 mMol) als Rh-Prekursor, 13.1 mg (S)-BINAP (0.021 mMol) als chiraler Diphosphinligand und 6 ml Toluol vorgelegt. Nach Ausschleusen wird die Lösung 30 Min. gerührt, dann werden 400 mg (R,S)-1-[3-Benzyloxy-6-hydroxy-2,4,5-trimethylphenethyl]-1-methylallylacetat (1.05mMol) als Substrat zugegeben und die gelb-orange Reaktionslösung 2 Stunden bei RT gerührt. Danach wird die Reaktionslösung 30 Min. ohne Schutzgas gerührt, eingedampft und das rote, ölige Rohprodukt an Kieselgel chromatographiert (Laufmittel : Hexan/Ether 3:1). Nach Eindampfen der gemäss Dünnschichtchromatographie einheitlichen Eluate und Trocknen der Rückstände werden 316 mg reines (S)-6-(Benzyloxy)-3,4-dihydro-2,5,7,8-tetramethyl-2-vinyl-2H-1-benzopyran als farbloses Oel isoliert, das beim Stehen erstarrt. Optische Reinheit : 44.8% , [α] = -26.3° .

Enantiomerenreines (S)-6-(Benzyloxy)-3,4-dihydro-2,5,7,8-tetramethyl-2-vinyl-2H-1-benzopyran kann durch einmalige Kristallisation gewonnen werden:
1.30 g (S)-6-(Benzyloxy)-3,4-dihydro-2,5,7,8-tetramethyl-2-vinyl-2H-1-benzopyran (o.p. 46.8%) werden bei RT in 6 ml Hexan und 1 ml Ether gelöst und 70h bei 5° kristallisieren gelassen. Nach Filtration werden 0.378 g optisch reines (S)-6-(Benzyloxy)-3,4-dihydro-2,5,7,8-tetramethyl-2-vinyl-2H-1-benzopyran. Smp. 63-66° , [α] = -59.07 erhalten.

Versuch 2

In zu Versuch 1 analoger Weise wird die Cyclisierung von 400 mg (R,S)-1-[3-Benzyloxy-6-hydroxy-2,4,5-trimethylphenethyl]-1-methylallylacetat in Anwesenheit von 9.8 mg $[Rh(COD)_2]^+PF_6^-$ als Rh-Precursor und 26.2 mg (S)-BINAP als Diphosphinliganden durchgeführt. Nach 16 Stunden bei 60° wird analog zu Versuch 1 aufgearbeitet, wobei man 162 mg reines (S)-6-(Benzyloxy)-3,4-dihydro-2,5,7,8-tetramethyl-2-vinyl-2H-1-benzopyran erhält. Optische Reinheit 10.6% , [α] = -6.2° .

Versuch 3

In zu Versuch 1 analoger Weise wird die Cyclisierung von 400 mg (R,S)-1-[3-Benzyloxy-6-hydroxy-2,4,5-trimethylphenethyl]-1-methylallylacetat bei 0° C durchgeführt. Nach 16 Stunden wird analog zu Versuch 1 aufgearbeitet, wobei man 224 mg reines (S)-6-(Benzyloxy)-3,4-dihydro-2,5,7,8-tetramethyl-2-vinyl-2H-1-benzopyran erhält. Optische Reinheit 36.8% , [α] = -21.6° .

Versuch 4

In zu Versuch 1 analoger Weise wird die Cyclisierung von 400 mg (R,S)-1-[3-Benzyloxy-6-hydroxy-2,4,5-trimethylphenethyl]-1-methylallylacetat in Anwesenheit von 12.4 mg (S)-BIPHEMP als chiralem Diphosphinliganden durchgeführt. Nach 2 Stunden wird analog zu Versuch 1 aufgearbeitet, wobei man 321 mg reines (S)-6-(Benzyloxy)-3,4-dihydro-2,5,7,8-tetramethyl-2-vinyl-2H-1-benzopyran erhält. Optische Reinheit 42.1% , [α] = -24.2° .

Versuch 4a

In zu Versuch 1 analoger Weise wird die Cyclisierung von 1.0 g (R,S)-1-[3-Benzyloxy-6-hydroxy-2,4,5-trimethylphenethyl]-1-methylallylacetat in Anwesenheit von 9.1 mg $[Rh(CF_3CO_2)(COD)]_2$ und von 20.9 mg (s)-mTolBINAP als chiralem Diphosphinliganden durchgeführt. Nach 2 Stunden wird analog zu Versuch 1 aufgearbeitet, wobei man 885 mg reines (S)-6-(Benzyloxy)-3,4-dihydro-2,5,7,8-tetramethyl-2-vinyl-2H-1-benzopyran erhält. Optische Reinheit 42.9%, [α] = -25.2° .

Versuch 4b

In zu Versuch 1 analoger Weise wird die Cyclisierung von 1.0 g (R,S)-1[3-Benzyloxy-6-hydroxy-2,4,5-trimethylphenethyl]-1-methylallylacetat in Anwesenheit von 9.1 mg $[Rh(CF_3CO_2)(COD)]_2$ und von 20.9 mg (S)-pTolBINAP als chiralem Diphosphinliganden durchgeführt. Nach 2h wird analog zu Versuch 1 aufgearbeitet, wobei man 881 mg reines (S)-6-(Benzyloxy)-3,4-dihydro-2,5,7,8-tetramethyl-2-vinyl-2H-1-benzopyran erhält. Optische Reinheit 34.8%, [α] = -20.4° .

Versuch 4c

In zu Versuch 1 analoger Weise wird die Cylisierung von 1.0 g (R,S)-1-[3-Benzyloxy-6-hydroxy-2,4,5-trimethylphenethyl]-1-methylallylacetat in Anwesenheit von 9.1 mg $[Rh(CF_3CO_2)(COD)]_2$ und von 18.9 mg (S)-mTolBIPHEMP als chiralem Diphosphinliganden durchgeführt. Nach 2 Stunden wird analog zu Versuch 1 aufgearbeitet, wobei man 888 mg reines (S)-6-(Benzyloxy-3,4-dihydro-2,5,7,8-tetramethyl-2-vinyl-2H-1-benzopopyran erhält. Optische Reinheit 55.0%, [α] = -32.3° .

Versuch 4d

In zu Versuch 1 analoger Weise wird die Cyclisierung von 1.0 g (R,S)-1-[3-Benzyloxy-6-hydroxy-2,4,5-trimethylphenethyl]-1-methylallylacetat in Anwesenheit von 9.1 mg $[Rh(CF_3CO_2)[COD)]_2$ und von 18.9 mg (S)-mTolBIPHEMP als chiralem Diphosphinliganden bei 0° C durchgeführt. Nach 3 Stunden wird analog zu Versuch 1 aufgearbeitet, wobei man 870 mg reines (S)-6-(Benzyloxy)-3,4-dihydro-2,5,7,8-tetramethyl-2-vinyl-2H-1-benzopyran erhält. Optische Reinheit 56.6%, [α] = -33.2° .

Versuch 4e

In zu Versuch 1 analoger Weise wird die Cyclisierung von 1.0 g (R,S)-1-[3-Benzyloxy-6-hydroxy-2,4,5-trimethylphenethyl]-1-methylallylacetat in Anwesenheit von 9.1 mg $[Rh(CF_3CO_2)(COD)]_2$ und von 18:9 mg (S)-pTolBIPHEMP als chiralem Diphosphinliganden durchgeführt. Nach 2 Stunden wird analog zu Versuch 1 aufgearbeitet, wobei man 882 mg reines (S)-6-(Benzyloxy)-3,4-dihydro-2,5,7,8-tetramethyl- 2-vinyl-2H-1-benzopyran erhält. Optische Reinheit 28.6%, [α] = -16.8° .

Versuch 4f

In zu Versuch 1 analoger Weise wird die Cyclisierung von 1.0 g (R,S)-1-[3-Benzyloxy-6-hydroxy-2,4,5-trimethylphenethyl]-1-methylallylacetat in Anwesenheit von 9.1 mg $[Rh(CF_3CO_2)(COD)]_2$ und von 18.1 mg (S)-MeOBIPHEP als chiralem Diphosphinliganden durchgeführt. Nach 2 Stunden wird analog zu Versuch 1 aufgearbeitet, wobei man 876 mg reines (S)-6-(Benzyloxy-3,4-dihydro-2,5,7,8-tetramethyl-2-vinyl-2H-1-benzopyran erhält. Optische Reinheit 35.6%, [α] = -20.9° .

Versuch 5

In einer Dry-Box ($O_2$-Gehalt < 1ppm) werden in einem 25 ml Glasautoklaven 5.2 mg $[RhCl(COD)]_2$ - (0.0105 mMol) als Rh-Precursor, 13.1 mg (S)-BINAP (0.021 mMol) als chiraler Diphosphinligand und 6 ml Toluol vorgelegt. Nach Aufdrucken von 15 bar Wasserstoff wird der Autoklav 2 Stunden bei RT geschüttelt. Nach dem Entspannen der Gase wird die dunkelrote Katalysatorlösung in ein 25 ml-Schlenkrohr vorgelegt. 400 mg (R,S)-1-[3-Benzyloxy-6-hydroxy-2,4,5-trimethyl-phenethyl]-1-methylallyl acetat (1.05mMol) werden zugegeben und die gelb-orange Reaktionslösung 18 Stunden bei 80° C gerührt. Nach zu Versuch 1 analoger Aufarbeitung werden 239 mg reines (S)-6-(Benzyloxy)-3,4-dihydro-2,5,7,8-tetramethyl-2-vinyl-2H-1-benzopyran isoliert. Optische Reinheit : 45.1% , [α] = -26.5° .

b) SUBSTRAT Typ IIa : (R,S)-1-[3-Benzyloxy-6-hydroxy-2,4,5-trimethylphenethyl]-1-methylallyl methylcarbonat

Versuch 6

In zu Versuch 1 analoger Weise wird die Cyclisierung von 3.34 g (R,S)-1-[3-Benzyloxy-6-hydroxy-2,4,5-trimethylphenethyl]-1-methylallyl methylcarbonat in Anwesenheit von 27.2 mg $[Rh(CF_3COO)(COD)]_2$ als Rh-Precursor und 57.6 mg (S)-BINAP als Diphosphinliganden durchgeführt. Nach 2 Stunden bei RT wird analog zu Versuch 1 aufgearbeitet, wobei man 2.07 g reines (S)-6-(Benzyloxy)-3,4-dihydro-2,5,7,8-tetramethyl-2-vinyl-2H-1-benzopyran erhält. Optische Reinheit 42.7% , $[\alpha] = -25.1^{\circ}$ .

Versuch 6a

In zu Versuch 1 analoger Weise wird die Cyclisierung von 1.0 g (R,S)-1-[3-Benzyloxy-6-hydroxy-2,4,5-trimethylphenethyl]-1-methylallyl methylcarbonat in Anwesenheit von 9.1 mg $[Rh(CF_3COO)(COD)]_2$ und 18.9 mg (S)-mTolBIPHEMP als Diphosphinliganden durchgeführt. Nach 2 Stunden bei RT wird analog zu Versuch 1 aufgearbeitet, wobei man 792 mg reines (S)-6-(Benzyloxy-3,4-dihydro-2,5,7,8-tetramethyl-2-vinyl-2H-1-benzopyran erhält. Optische Reinheit 43.4%, $[\alpha] = -25.5^{\circ}$ .

Versuch 6b

In zu Versuch 1 analoger Weise wird die Cyclisierung von 1.0 g (R,S)-1-[3-Benzyloxy-6-hydroxy-2,4,5-trimethylphen ethyl]-1-methylallyl methylcarbonat in Anwesenheit von 9.1 mg $[Rh(CF_3COO)(COD)_2$ und 18.9 mg (S)-pTolBIPHEMP als Diphosphinliganden durchgeführt. Nach 2 Stunden bei RT wird analog zu Versuch 1 aufgearbeitet, wobei man 716 mg reines (S)-6-Benzyloxy)-3,4-dihydro-2,5,7,8-tetramethyl-2-vinyl-2H-1-benzopyran erhält. Optische Reinheit 39.5%, $[\alpha] = 23.2^{\circ}$ .

Versuch 6c

In zu Versuch 1 analoger Weise wird die Cyclisierung von 1.0 g (R,S)-1-[3-Benzyloxy-6-hydroxy-2,4,5-trimethylphen ethyl]-1-methylallyl methylcarbonat in Anwesenheit von 9.1 mg $[Rh(CF_3COO)(COD)]_2$ und 18.1 mg (S)-MeOPBIPHEP als Diphosphinliganden durchgeführt. Nach 2 Stunden bei RT wird analog zu Versuch 1 aufgearbeitet, wobei man 502 mg reines (S)-6-Benzyloxy)-3,4-dihydro-2,5,7,8-tetramethyl-2-vinyl-2H-1-benzopyran erhält. Optische Reinheit 40.2%, $[\alpha] = -23.6^{\circ}$ .

Versuch 7

In einer Dry-Box ($O_2$-Gehalt < 1ppm) werden in einem Kolben 34.1 mg $[Rh(COD)_2]^+ BF_4^-$ (0.084 mMol) und 57.4 mg (S)-BINAP (0.092 mMol) in 32 ml Toluol vorgelegt. Nach 2 Stunden Rühren werden 31.4 mg Tetrabutylammoniumtrifluoracetat (0.088 mMol) und 4 ml Toluol zugegeben und die Suspension gerührt, bis man eine rot-orange Lösung erhält (22-24h). Danach wird eine Lösung von 3.35 g (R,S)-1-[3-Benzyloxy-6-hydroxy-2,4,5-trimethylphenethyl] -1-methylallyl methylcarbonat in 12 ml Toluol zugegeben und die orange Lösung 2 Stunden gerührt. Danach wird analog zu Versuch 1 aufgearbeitet, wobei man 2.06 g reines (S)-6-(Benzyloxy)- 3,4-dihydro-2,5,7,8-tetramethyl-2-vinyl-2H-1-benzopyran erhält. Optische Reinheit : 37.1% , $[\alpha] = -21.8^{\circ}$ .

Versuch 8

In einer Dry-Box ($O_2$-Gehalt < 1ppm) werden in einem Kolben 7.2 mg [Rh(2-Methylallyl)(COD)] (0.0288 mMol) und 19.4 mg (S)-BINAP (0.031 mMol) in 17 ml Toluol vorgelegt. Nach 30 Min. Rühren werden 3.2 mg Trifluoressigsäure (0.0281 mMol) als Lösung in 1 ml Toluol und nach weiteren 30 Min. 1.12 g (R,S)-1-[3-Benzyloxy-6-hydroxy-2,4,5-trimethyl-phenethyl]-1-methylallyl methylcarbonat zugegeben. Nach 3 Stunden wird analog zu Versuch 1 aufgearbeitet, wobei man 0.70 g reines (S)-6-(Benzyloxy)-3,4-dihydro-2,5,7,8-tetra-methyl-2-vinyl-2H-1-benzopyran erhält. Optische Reinheit : 40.4% , [α] = -23.8° .

Versuch 9

In zu Versuch 7 analoger Weise wird die Cyclisierung von 3.35 g (R,S)-1-[3-Benzyloxy-6-hydroxy-2,4,5-trimethylphenethyl]-1-methylallyl methylcarbonat (8.4 mMol) in Anwesenheit von 8.5 mg $[Rh(COD)_2]^+ BF_4^-$ (0.021 mMol) und 7.8 mg Tetrabutylammonium-trifluoracetat (0.022 mMol) durchgeführt. Nach 5.5 Stunden wird analog zu Versuch 1 aufgearbeitet, wobei man 1.94 g reines (S)-6-(Benzyloxy)-3,4-dihydro-2,5,7,8-tetramethyl-2-vinyl-2H-1-benzopyran erhält. Optische Reinheit : 36.6% , [α] = -21.5° .

Versuch 10

In zu Versuch 5 analoger Weise werden einer Lösung von 3.4 mg $[Rh(CF_3COO)(COD)]_2$ (0.0052 mMol) und 7.2 mg (S)-BINAP in 6ml Toluol nach Vorhydrierung mit 0.42g (R,S)-1-[3-Benzyloxy-6-hydroxy-2,4,5-trimethylphenethyl]-1-methylallyl methylcarbonat versetzt. Nach 2 Stunden wird analog zu Versuch 1 aufgearbeitet, wobei man 0.33 g reines (S)-6-(Benzyloxy)-3,4-dihydro-2,5,7,8-tetramethyl-2-vinyl-2H-1-benzopyran erhält. Optische Reinheit : 37.4% , [α] = -22.0° .

Versuch 11

In zu Versuch 1 analoger Weise wird die Cyclisierung von 418 mg (R,S)-1-(3-Benzyloxy-6-hydroxy-2,4,5-trimethylphenethyl]-1-methylallyl methylcarbonat in Anwesenheit von 6.8 mg $[Rh(CF_3COO)(COD)]_2$ und 13.1 mg (S)-BINAP in 6 ml Methylenchlorid durchgeführt. Nach 1.5 Stunden wird analog zu Versuch 1 aufgearbeitet, wobei man 250 mg reines (S)-6-(Benzyloxy)-3,4-dihydro-2,5,7,8-tetramethyl-2-vinyl-2H-1-benzopyran erhält. Optische Reinheit : 38.1% , [α] = -22.4° .

Versuch 12

In zu Versuch 1 analoger Weise wird die Cyclisierung von 418 mg (R,S)-1-(3-Benzyloxy-6-hydroxy-2,4,5-trimethylphenethyl]-1-methylallyl methylcarbonat in Anwesenheit von 6.8 mg $[Rh(CF_3COO)(COD)]_2$ und 13.1 mg (R)-BINAP in 6 ml Tetrahydrofuran durchgeführt. Nach 1.5 Stunden wird analog zu Versuch 1 aufgearbeitet, wobei man 80 mg reines (S)-6-(Benzyloxy)-3,4-dihydro-2,5,7,8-tetramethyl-2-vinyl- 2H-1-benzopyran erhält. Optische Reinheit : 19.9% , [α] = -11.7° .

Versuch 13

In zu Versuch 1 analoger Weise wird die Cyclisierung von 418 mg (R,S)-1-[3-Benzyloxy-6-hydroxy-2,4,5-trimethylphenethyl]-1-methylallyl methylcarbonat in Anwesenheit von 6.8 mg $[Rh(CF_3COO)(COD)]_2$ und 104.8 mg (S)-BINAP in 6 ml Toluol durchgeführt. Nach 6.5 Stunden wird analog zu Versuch 1 aufgearbeitet, wobei man 227 mg reines (S)-6-(Benzyloxy)-3,4-dihydro-2,5,7,8-tetramethyl-2-vinyl-2H-1-benzopyran erhält. Optische Reinheit : 46.0% , [α] = -27.0° .

Versuch 14

In zu Versuch 1 analoger Weise wird die Cyclisierung von 1.11 g (R,S)-1-[3-Benzyloxy-6-hydroxy-2,4,5-trimethylphenethyl]-1-methylallyl methylcarbonat (2.9 mMol) in Anwesenheit von 7.1 mg $[RhCl(COD)]_2$ -(0.014 mMol) und 19.5 mg (S)-BINAP (0.030 mMol) durchgeführt. Nach 24 Stunden wird analog zu Versuch 1 aufgearbeitet, wobei man 0.685 g reines (S)-6-(Benzyloxy)-3,4-dihydro-2,5,7,8-tetramethyl-2-vinyl-2H-1-benzopyran erhält. Optische Reinheit : 41.4%, [α] = -24.3° .

Versuch 15

In zu Versuch 1 analoger Weise wird die Cyclisierung von 1.11 g (R,S)-1-(3-Benzyloxy-6-hydroxy-2,4,5-trimethylphenethyl]-1-methylallyl methylcarbonat (2.9 mMol) in Anwesen heit von 13.2 mg $(Rh(COD)_2]^+$ $PF_6^-$ (0.028 mMol) und 36.7 mg (S)-BINAP (0.059 mMol) durchgeführt. Nach 64 Stunden wird analog zu Versuch 1 aufgearbeitet, wobei man 0.374g reines (S)-6-(Benzyloxy)-3,4-dihydro-2,5,7,8-tetra-methyl-2-vinyl-2H-1-benzopyran erhält. Optische Reinheit : 24.4% , [α] = -14.4° .

Versuch 16

In zu Versuch 7 analoger Weise wird die Cyclisierung von 418 mg (R,S)-1-[3-Benzyloxy-6-hydroxy-2,4,5-trimethylphenethyl]-1-methylallyl methylcarbonat (1.05 mMol) in Anwesenheit von 9.9 mg $(Rh(COD)_2]^+$ $PF_6^-$ (0.021 mMol), 14.5 mg (S)-BINAP (0.023 mMol) und 6.2 mg Tetraethylammoniumacetat tetrahydrat (0.024 mMol) in 9 ml Toluol durchgeführt. Nach 6 Stunden wird analog zu Versuch 1 aufgearbeitet, wobei man 192 mg reines (S)-6-(Benzyloxy)-3,4-dihydro-2,5,7,8-tetramethyl-2-vinyl-2H-1-benzopyran erhält. Optische Reinheit : 37.4% , [α] = -22.0° .

Versuch 17

In zu Versuch 8 analoger Weise wird die Cyclisierung von 1.12 g (R,S)-1-[3-Benzyloxy-6-hydroxy-2,4,5-trimethylphenethyl]-1-methylallyl methylcarbonat (2.80 mMol) in Anwesenheit von 7.2 mg [Rh(2-Methylallyl)-(COD)] (0.0288 mMol), 19.4 mg (S)-BINAP (0.031 mMol) und 1.68 mg Essigsäure (0.028 mMol) in 17 ml Toluol durchgeführt. Nach 24 Stunden wird analog zu Versuch 1 aufgearbeitet, wobei man 0.36 g reines (S)-6-(Benzyloxy)-3,4-dihydro-2,5,7,8-tetramethyl-2-vinyl-2H-1-benzopyran erhält. Optische Reinheit : 35.4% , [α] = -20.8° .

Versuch 18

In zu Versuch 7 analoger Weise wird die Cyclisierung von 3.35 g (R,S)-1-[3-Benzyloxy-6-hydroxy-2,4,5-trimethylphenethyl]-1-methylallyl methylcarbonat in Anwesenheit von 34.0 mg $[Rh(COD)_2]^+PF_6^-$ als Rh-Precursor, 51.0 mg (S)-BIPHEMP als Diphosphinligand und 31.3 mg Tetrabutylammoniumtrifluoracetat durchgeführt. Nach 2 Stunden bei RT wird analog zu Versuch 1 aufgearbeitet, wobei man 2.35 g (S)-6-(Benzyloxy)-3,4-dihydro-2,5,7,8-tetramethyl-2-vinyl-2H-1-benzopyran erhält. Optische Reinheit 28.3% , [α] = -16.6° .

Versuch 19

In zu Versuch 1 analoger Weise wird die Cyclisierung von 3.35 g (R,S)-1-[3-Benzyloxy-6-hydroxy-2,4,5-trimethylphenethyl]-1-methylallyl methylcarbonat in Anwesenheit von 27.3 mg $[Rh(CF_3COO)(COD)]_2$ als Rh-Precursor und 57.5 mg (S)-BINAP als Diphosphinliganden bei 0°C durchgeführt. Nach 24 Stunden wird analog zu Versuch 1 aufgearbeitet, wobei man 1.93 g (S)-6-(Benzyloxy)-3,4-dihydro-2,5,7,8-tetramethyl-2-vinyl-2H-1-benzopyran erhält. Optische Reinheit 61.8% , [α] = -36.3° .

Versuch 20

In zu Versuch 7 analoger Weise wird die Cyclisierung von 3.35 g (R,S)-1-[3-Benzyloxy-6-hydroxy-2,4,5-trimethylphenethyl]-1-methylallyl methylcarbonat in Anwesenheit von 39.2 mg $[Rh(COD)_2]^+PF_6^-$ als Rh-Precursor, 57.5 mg (S)-BINAP als Diphosphinliganden und 48.0 mg Tetrabutylammoniumtrifluoracetat bei 0°C durchgeführt. Nach 24 Stunden wird analog zu Versuch 1 aufgearbeitet, wobei man 1.72 g (S)-6-(Benzyl oxy)-3,4-dihydro-2,5,7,8-tetramethyl-2-vinyl-2H-1-benzopyran erhält. Optische Reinheit 58% , [α] = -33.8° .

Versuch 21

In zu Versuch 1 analoger Weise wird die Cyclisierung von 418 mg (R,S)-1-(3-Benzyloxy-6-hydroxy-2,4,5-trimethylphenethyl]-1-methylallyl methylcarbonat in Anwesenheit von 6.8 mg $[Rh(CF_3COO)(COD)]_2$ als Rh-Precursor und 11.6 mg (S)-BIPHEMP als Diphosphinliganden bei 0°C durchgeführt. Nach 24 Stunden wird analog zu Versuch 1 aufgearbeitet, wobei man 160 mg (S)-6-(Benzyloxy)-3,4-dihydro-2,5,7,8-tetramethyl-2-vinyl-2H-1-benzopyran erhält. Optische Reinheit 52% , [α] = -30.5° .

Versuch 22

In zu Versuch 1 analoger Weise wird die Cyclisierung von 3.35 g (R,S)-1-[3-Benzyloxy-6-hydroxy-2,4,5-trimethylphenethyl]-1-methylallyl methylcarbonat in Anwesenheit von 27.7 mg $(Rh(CF_3COO)(COD)]_2$ als Rh-Precursor und 57.6 mg (S)-BINAP als Diphosphinliganden in wassergesättigtem Toluol durchgeführt. Nach 2 Stunden bei RT wird analog zu Versuch 1 aufgearbeitet, wobei man 1.10 g (S)-6-(Benzyloxy)-3,4-dihydro-2,5,7,8-tetramethyl-2-vinyl-2H-1-benzopyran erhält. Optische Reinheit 73.6% , [α] = -43.2° .

Versuch 23

In zu Versuch 1 analoger Weise wird die Cyclisierung von 1.12 g (R,S)-1-[3-Benzyloxy-6-hydroxy-2,4,5-trimethylphen ethyl]-1-methylallyl methylcarbonat in Anwesenheit von 6.9 mg $(RhC_1(COD)]_2$ als Rh-Precursor und 19.2 mg (S)-BINAP als Diphosphinliganden in wassergesättigtem Toluol durchgeführt. Nach 24 Stunden bei RT wird analog zu Versuch 1 aufgearbeitet, wobei man 252 mg (S)-6-(Benzyloxy)-3,4-dihydro-2,5,7,8-tetramethyl-2-vinyl-2H-1-benzopyran erhält. Optische Reinheit 65.7% , [α] = -38.6° .

Versuch 24

In zu Versuch 7 analoger Weise wird die Cyclisierung von 3.35 g (R,S)-1-[3-Benzyloxy-6-hydroxy-2,4,5-trimethylphenethyl]-1-methylallyl methylcarbonat in Anwesenheit von 34.1 mg $[Rh(COD)_2]^+BF_4^-$ als Rh-Precursor, 57.4 mg (S)-BINAP als Diphosphinliganden und 31.3 mg Tetrabutylammoniumtrifluoracetat in wassergesättigtem Toluol durchgeführt. Nach 2 Stunden bei RT wird analog zu Versuch 1 aufgearbeitet, wobei man 1.23 g (S)-6-(Benzyloxy)-3,4-dihydro-2,5,7,8-tetramethyl-2-vinyl-2H-1-benzopyran erhält. Optische Reinheit 77.6% , [α] = -45.6° .

Versuch 25

In zu Versuch 8 analoger Weise wird die Cyclisierung von 1.12 g [R,S)-1-[3-Benzyloxy-6-hydroxy-2,4,5-trimethylphenethyl]-1-methylallyl methylcarbonat in Anwesenheit von 7.0 mg [Rh(Methallyl)(COD)] als Rh-Precursor, 19.2 mg (S)-BINAP als Diphosphinliganden und 3.2 mg Trifluoressigsäure in wassergesättigtem Toluol durchgeführt. Nach 3 Stunden bei RT wird analog zu Versuch 1 aufgearbeitet, wobei man 0.35 g (S)-6-(Benzyloxy)-3,4-dihydro-2,5,7,8-tetramethyl-2-vinyl-2H-1-benzopyran erhält. Optische Reinheit 76.9% ,[α] = -45.2° .

Versuch 26

In zu Versuch 7 analoger Weise wird die Cyclisierung von 3.35 g (R,S)-1-[3-Benzyloxy-6-hydroxy-2,4,5-trimethylphenethyl]-1-methylallyl methylcarbonat in Anwesenheit von 34.1 mg $[Rh(COD)_2]^+BF_4^-$ als Rh-Precursor, 50.8 mg (S)-BIPHEMP als Diphosphinliganden und 31.3 mg Tetrabutylammoniumtrifluoracetat in wassergesättigtem Toluol durchgeführt. Nach 2.4 Stunden bei RT wird analog zu Versuch 1 aufgearbeitet, wobei man 1.40 g (S)-6-(Benzyloxy)-3,4-dihydro-2,5,7,8-tetramethyl-2-vinyl-2H-1-benzopyran erhält. Optische Reinheit 67.9% ,[α] = -39.9° .

<u>Cyclisierungsversuche in tabellarischer Darstellung</u>

Asymmetrische Cyclisierung verschiedener Allylalkoholderivate der Formel II.

Versuchsdurchführung analog zu Versuch 1

| Vers.No | Substrat -Typ | R° | W | Ausbeute [%] | Vinylchroman I [α] | Vinylchroman I o.R. [%] |
|---|---|---|---|---|---|---|
| 1 | IIa | Bz | OAc | 95 | -26.3 | 44.8 |
| 6 | " | " | $OCO_2$-Me | 76 | -25.1 | 42.7 |
| 27 | " | " | $OCO_2$-t-Bu | 81 | -21.0 | 35.7 |
| 28 | " | Me | OAc | 86 | -32.9 | 42.5 |

Cyclisierungsversuche in tabellarischer Darstellung (Fortsetzung)

Asymmetrische Cyclisierung verschiedener Allylalkoholderivate der Formel II.

Versuchsdurchführung analog zu Versuch 1

| Vers.No | Substrat-Typ | R° | W | Vinylchroman I: Ausbeute [%] | [α] | o.R. [%] |
|---|---|---|---|---|---|---|
| 29 | " | " | $OCO_2$-Me | 70 | -37.7 | 48.7 |
| 30 | " | " | $OCO_2$-t-Bu | 74 | -25.1 | 32.5 |
| 31 | " | " | $OCO_2$-Bz | 56 | -27.6 | 35.7 |
| 32 | " | Ac | $OCO_2$-Me | 70 | -31.1 | 39.1 |
| 33 | IIb | Bz | $OCO_2$-Me | 42 | -9.8 | 16.7 |

C. Hydroborierung-Oxidation von I zu VI

Beispiel 4

Diese Reaktion wird in Anlehnung an H.C.Brown, J.C.Chen, J.Org.Chem., 46, 3988 (1981) durchgeführt.

Eine Lösung von 1.22 g 9-Borabicyclo[3.3.1]nonan (10.0 mMol) in Tetrahydrofuran (50 ml) wird mit 2.67 g (R,S)-6-(Benzyloxy)-3,4-dihydro-2,5,7,8-tetramethyl-2-vinyl-2H-1-benzopyran (8.3 mMol) versetzt und 2 Stunden bei 50 °C gerührt. Nach Abkühlen des Reaktionsgemisches werden nacheinander Ethanol (15 mL), 6N Natronlauge (7.5 mL) und 30%-iges Wasserstoffperoxyd (7.5 mL) zugegeben und 3 Stunden bei 50 °C

reagieren gelassen. Nach Abkühlen wird das Reaktions-gemisch mit Kaliumcarbonat gesättigt und mit Ether extrahiert. Die vereinigten organischen Extrakte werden getrocknet und eingedampft. Der verbleibende Rückstand wird an Kieselgel gereinigt (Laufmittel : Hexan/Ether 1:2) und das reine Produkt aus Ether/Hexan kristallisiert. Man erhält 2.57g (91.1%) weisse Kristalle, Smp.: 67.5-68.5 .

D. Herstellung der prochiralen Substrate (Typ IIb, IIc)

## Beispiel 5

a) Eine Lösung von Chlormethyltrimethylhydrochinon-dimethyläther (7,5 g; 32 mMol) in THF (20 ml) wird bei 0° tropfenweise mit einer frisch bereiteten Lösung eines Grignard-reagenses, hergestellt aus 1.9 g Magnesiumspänen und 4,91 ml (65 mMol) Propargylbromid versetzt. Nach Zugabe von festem Natriumjodid (0,42 g) wird auf Raumtemperatur erwärmt und über Nacht weitergerührt. Danach wird das Reaktionsgemisch mit Aether verdünnt und mit 2N Salzsäure gewaschen. Die ätherische Lösung wird mit Bicarbonat entsäuert, getrocknet und vom Lösungsmittel befreit. Destillation (0,04 mbar; 140° C) des verbleibenden Rückstandes ergibt 7,16 g 1-(3-Butynyl)-3,6-dimethoxy-2,4,5-trimethylbenzol mit einem Schmelzpunkt vom 54° C. IR: 3308, 2824, 2115, 1265, 1245 $cm^{-1}$. MS: 232 ($M^+$), 193 ($-CH_2C\equiv CH$).

b) Eine Suspension von Zirkonocen-dichlorid (2,61 g; 8,9 mMol) in 1,2-Dichloräthan wird zunächst mit Trimethylaluminium (10,9 ml; 114 mMol), dann mit einer Lösung von 1-(3-Butynyl)-3,6-dimethoxy-2,4,5-trimethylbenzol (10,6 g; 45,7 mMol) in 1,2-Dichloräthan (60 ml) versetzt. Es wird 46 Stunden auf 50° C erwärmt, danach auf -40° gekühlt, mit THF (20 ml) verdünnt, mit 40 ml einer 1.6 molaren n-Butyllithiumlösung (in Hexan) und schliesslich mit 4.37 g Paraformaldehyd behandelt. Das entstehende Reaktionsgemisch wird 20 Stunden bei Raumtemperatur nachgerührt und anschliessend in eiskalte 2N Salzsäurelösung eingetragen. Es wird zweimal mit Aether extrahiert, und die vereinigten Extrakte werden mit Wasser gewaschen und getrocknet. Das nach Entfernen des Lösungsmittels verbleibende Oel wird an Kieselgel (Laufmittel: Hexan/Essigester = 2.5/1) gereinigt. Man erhält (E)-5-(2,5-Dimethoxy-3,4,6-trimethylphenyl)-3-methyl -2-penten-1-ol als farbloses Oel, das langsam bei Raumtemperatur kristallisiert (Smp: 46° C). Die Substanz zeigt IR-Banden bei 3350, 1668 und 1245 $cm^{-1}$ und ein Massenspektrum mit Peaks bei 278 ($M^+$), 260 ($-H_2O$) und 193 ($-(CH_3)_2C=CHCH_2OH$) Masseneinheiten.

c) Eine Lösung von 4,17 g (15 mMol) (E)-5-(2,5-Dimethoxy-3,4,6-trimethylphenyl)-3-methyl -2-penten-1-ol in Pyridin (10 ml) und Essigsäureanhydrid (2 ml) wird 5 Stunden bei Raumtemperatur gerührt und danach in eiskalte 2N Salzsäurelösung gegossen. Es wird mit Aether extrahiert. Die vereinigten Extrakte werden mit Wasser gewaschen, getrocknet, vom Lösungsmittel befreit; der so erhaltene Rückstand wird an Kieselgel mit Hexan/Essigester (95:5) gereinigt. Man erhält 3,8 g (E)-5-(2,5-Dimethoxy-3,4,6-trimethylphenyl)-3-methyl-2-pentenylacetat als farbloses Oel. $^1H$-NMR ($CDCl_3$, δ): 1,60 (s,3H,$CH_3C=$), 2,07 (s,3H,Ac), 2,18 (s,6H), 2.21 (s,3H,$CH_3$-Ar), 3,64 und 3,68 (s,3H,$CH_3O$), 4,62 (d,2H,$CH_2O$), 5,43 (t,1H,$CH=C$).

d) Eine Lösung von 3,83 g (12 mMol) (E)-5-(2,5-Dimethoxy-3,4,6-trimethylphenyl)-3-methyl -2-pentenylacetat in Acetonitril (50 ml) wird tropfenweise bei 0° C mit einer Lösung von Cerammoniumnitrat (16,4 g; 30 mMol) in Wasser (50 ml) versetzt. Nach beendeter Zugabe wird noch 20 Minuten nachgerührt und mit Wasser und Chloroform verdünnt. Die organische Phase wird abgetrennt, mit Wasser gewaschen, getrocknet und zu einem gelben Oel konzentriert. Das so erhaltene (E)-3-Methyl-5-(2,4,5-trimethyl-3,6-dioxo-1,4-cyclohexadien -1-yl)-2-pentenylacetat (3,27 g) weist im IR-Spektrum Banden bei 1738, 1641 und 1233 $cm^{-1}$ sowie im Massenspektrum Banden bei 290 ($M^+$), 248 ($-C_2H_2O$), 231 ($-C_2H_2O-H_2O$) und 215 (230-$CH_3$) auf.

e) Eine Lösung von 3,27 g (11,3 mMol) (E)-3-Methyl-5-(2,4,5-trimethyl-3,6-dioxo-1,4-cyclohexadien -1-yl)-2-pentenylacetat in 60 ml THF wird tropfenweise mit 40 ml einer frisch bereiteten 0,5M Natriumdithionit-lösung behandelt. Nach beendeter Zugabe wird mit Wasser verdünnt und mit Chloroform extrahiert. Die vereinigten Extrakte werden mit Wasser gewaschen und vom Lösungsmittel befreit. Der feste Rückstand wird mit Hexan gewaschen und an der Oelpumpe getrocknet. Es verbleiben 2,94 g (E)-5-(2,5-Dihydroxy-3,4,6-trimethylphenyl)-3-methyl -2-pentenylacetat als farbloses Pulver, Smp. 94,5-96° C. Das so erhaltene Material absorbiert im infraroten Bereich bei 3400, 1726, 1667 und 1635 $cm^{-1}$ und weist im Massenspektrum Banden bei 292 ($M^+$), 232 (-AcOH) und 164 ($-CH_2(CH_3)C=CHCH_2OAc$) auf.

a1) Eine Lösung von 1 g (3,6 mMol) (E)-5-(2,5-Dimethoxy-3,4,6-trimethylphenyl)-3-methyl -2-penten-1-ol in Pyridin (10 ml) wird in drei Portionen mit insgesamt 1,08 ml Chlorameisensäuremethylester versetzt und das entstehende Gemisch über Nacht nachgerührt. Danach wird auf Eis gegossen und mit Essigester

extrahiert. Die Extrakte werden mit Wasser gewaschen, getrocknet, eingeengt und der verbleibende Rückstand wird an Kieselgel mit Hexan/Essigester (9:1) gereinigt. Es verbleiben 0,8 g (E)-5-(3,6-Dimethoxy-2,4,5-trimethylphenyl)-3-methyl -2-pentenylmethylcarbonat als farbloses Oel. IR: 2941, 1748, 1666 und 1265 $cm^{-1}$. MS: 336 ($M^+$), 260 (-MeOH-$CO_2$).

b1) Eine Lösung von 3,8 g (6,9 mMol) Cerammoniumnitrat in 15 ml Wasser wird zu einer auf 0° C gekühlen Lösung von 0.93 g (2,8 mMol) (E)-5-(3,6-Dimethoxy-2,4,5-trimethylphenyl)-3-methyl -2-pentenylmethylcarbonat in Acetonitril (15 ml) getropft. Es wird 15 Minuten nachgerührt und danach zweimal mit je 100 ml Chloroform extrahiert. Die vereinigten Extrakte werden mit Wasser gewaschen, getrocknet und vom Lösungsmittel befreit. Der verbleibende Rückstand wird an Kieselgel mit Hexan/Essigester (9:1) chromatographiert. Man erhält 0,73 g (E)-3-Methyl-5-(2,4,5-trimethyl -3,6-dioxo-1,4-cyclohexadien-1-yl)-2-pentenylmethylcarbonat als hellgelbes Oel. $^1$H-NMR ($CDCl_3$ $\delta$): 1,65 (s,3H), 2,01 (s,9H), 2,1 und 2,60 (m,2H), 3,77 (s,3H), 4,63 (d,2H), 5,38 (t,1H).

c1) Eine frisch bereitete 0,5M Natriumdithionitlösung (7,2 ml) wird tropfenweise zu einer Lösung von (E)-3-Methyl-5-(2,4,5-trimethyl-3,6-dioxo-1,4-cyclohexadien -1-yl)-2-pentenylmethylcarbonat (0,73 g; 2,4 mMol) in THF (1 ml) zugegeben. Nach beendeter Zugabe wird mit Wasser verdünnt und mit Methylenchlorid (2 x50 ml) extrahiert. Die vereinigten Extrakte werden mit Wasser gewaschen, getrocknet und vom Lösungsmittel befreit. Der beigefarbene Rückstand wird erst mit Hexan/Methylenchlorid (6:1) (7 ml), dann mit Hexan (7 ml) gewaschen. Es verbleiben 0,63 g eines farblosen Pulvers vom Schmelzpunkt 101-102,5° C. Das so erhaltene (E)-5-(3,6-Dihydroxy-2,4,5-trimethylphenyl) -2-pentenylmethylcarbonat absorbiert im infraroten Bereich bei 3380, 1747, 1639 und 1269 $cm^{-1}$. Das Massenspektrum weist Banden bei 308 ($M^+$), 232 (-$CO_2$-MeOH), 217 (232-$CH_3$), 203 (232-$CH_2CH_3$) und 164 auf.

a2) Benzoylchlorid (1 g; 7,1 mMol) wird zusammen mit (E)-5-(2,5-Dimethoxy-3,4,6-trimethylphenyl) -3-methyl-2-penten-1-ol (1,56 g: 5,6 mMol) in Pyridin (10 ml) gelöst. Nach 30 Minuten wird auf Wasser gegossen und mit Aether extrahiert. Die Extrakte werden mit 1N Salzsäure, Wasser, gesättigter Bicarbonatlösung und wieder mit Wasser gewaschen. Nach dem Entfernen des Lösungsmittel im Vakuum verbleiben 2 g eines farbloses Oels. Das so erhaltene (E)-5-(2,5-Dimethoxy-3,4,6-trimethylphenyl)-3-methyl -2-pentenylbenzoat bedarf keiner weiteren Reinigung; es weist im IR-Spektrum Banden bei 2839, 1718, 1454 und 1270 $cm^{-1}$ auf.

b2) Eine Lösung von Cerammoniumnitrat (7,2 g; 13,1 mMol) in Wasser (30 ml) wird unter Eiskühlung zu einer Lösung von (E)-5-(2,5-Dimethoxy-3,4,6-trimethylphenyl) -3-methyl-2-pentenylbenzoat (2 g; 5.2 mMol) in Acetonitril (30 ml) getropft. Nach beendeter Zugabe wird mit Wasser verdünnt und mit Chloroform extrahiert. Die Extrakte werden mit Wasser gewaschen, getrocknet und vom Lösungsmittel befreit. Der Rückstand kristallisiert spontan und man erhält 1,35 g (E)-3-Methyl-5-(2,4,5-trimethyl-3,6-dioxo-1,4 -cyclohexadien-1-yl)-2-pentenylbenzoat vom Schmelzpunkt 74,5-75,5° C. $^1$H-NMR ($CDCl_3$, $\delta$): 1,64 (s,3H), 1,99 (s,6H), 2,03 (s,3H), 2,13 und 2,62 (je dd,2H), 4,82 (d,2H), 5,45 (t,1H) und 7,43-8,05 (m,5H).

c2) Eine Lösung von 1,22 g (3,5 mMol) (E)-3-Methyl-5-(2,4,5-trimethyl-3,6-dioxo-1,4 -cyclohexadien-1-yl)-2-pentenylbenzoat in 10 ml THF wird tropfenweise mit einer Lösung von 0,9 g Natriumdithionit in 8 ml Wasser behandelt. Nach 1 Stunde wird mit Chloroform extrahiert (2 x) und die vereinigten Extrakte dreimal mit Wasser gewaschen. Der nach Entfernen des Lösungsmittels verbleibende Rückstand wird mit Hexan gewaschen und an der Oelpumpe getrocknet. Man erhält 1,05 g farbloses (E)-5-(2,5-Dihydroxy-3,4,6-trimethylphenyl)-3-methyl -2-pentenylbenzoat vom Schmelzpunkt 112-114° C. Es absorbiert im infraroten Bereich bei 3341, 1720, 1640, 1600, 1583, 1425 und 1278 $cm^{-1}$.

a3) Eine Lösung von 5 g (21,5 mMol) 1-(3-Butynyl)-3,6-dimethoxy-2,4,5-trimethylbenzol in 80 ml THF wird auf -78° C abgekühlt und zunächst mit 13,4 ml einer 1,6M n-Butyllithiumlösung, dann mit 2,2 g (23,3 mMol) Chlorameisensäuremethylester versetzt. Es wird 1 Stunde bei -78° C belassen, dann auf -20° C aufgewärmt. 2 Stunden bei dieser Temperatur gehalten und schliesslich auf gesättigte Ammoniumchloridlösung gegossen. Extraktion mit Essigester liefert nach Waschen der Extrakte mit Wasser und Trocknen über Natriumsulfat ein gelbes Oel, das an Kieselgel (Eluens: Hexan/Essigester 95:5→90:10) gereinigt wird. Man erhält 3,6 g spontan kristallisierenden 5-(2,5-Dimethoxy-3,4,6-trimethylphenyl)-2-pentinsäuremethylester vom Schmelzpunkt 71-72° C. $^1$H-NMR ($CDCl_3$, $\delta$): 1,61 (s,3H), 2,16, 2,17, 2,24 (je s,3H). 2,50 und 2,92 (je t,2H), 3,64, 3,67 und 3,76 (je s,3H).

b3) Eine Lösung von Lithiumdimethylcuprat (hergestellt aus 0,97 g (5,1 mMol) Kupfer-(I)-jodid und 6,4 ml einer 1,6M ätherischen Methyllithiumlösung) in Aether (30 ml) wird auf -78° C gekühlt und tropfenweise mit einer Lösung von 5-(2,5-Dimethoxy-3,4,6-trimethylphenyl) -2-pentinsäuremethylester (1 g; 3,4 mMol) in THF (10 ml) versetzt. Es wird 90 Minuten bei -78° C nachgerührt, mit 0,5 ml Methanol versetzt, auf Raumtemperatur erwärmt und mit gesättigter Ammoniumchloridlösung versetzt. Es wird zweimal mit Aether extrahiert und die vereinigten Extrakte werden mit Wasser gewaschen und getrocknet. Der nach Entfernen des Lösungsmittels verbleibende Rückstand wird an Kieselgel mit Hexan/Essigester

(95:5) gereinigt. Man erhält 0,81 g kristallinen (Z)-5-(2,5-Dimethoxy-3,4,6-trimethylphenyl)-3-methyl -2-pentensäuremethylester vom Schmelzpunkt 87,5-89° C. $^1$H-NMR ($CDCl_3$, δ): 1,99 (d,3H,J=0,1Hz), 2,16 (s,6H), 2,33 (s,3H), 2,77 (s,4H), 3,65, 3,68 und 3,72 (je s,3H), 5,70 (q,1H,J=0,1Hz).

c3) Eine Lösung von 0,8 g (2,6 mMol) (Z)-5-(2,5-Dimethoxy-3,4,6-trimethylphenyl)-3-methyl -2-pentensäuremethylester in Toluol (5 ml) wird tropfenweise mit 0,75 ml einer 3,5 molaren Red-Al-Lösung in Toluol behandelt. Nach 2 Stunden bei Raumtemperatur wird auf gesättigte Ammoniumchloridlösung gegossen und mit Aether extrahiert. Die vereinigten Extrakte werden mit Wasser gewaschen. getrocknet und zu einem farblosen Oel konzentriert, das an Kieselgel mit Hexan/Essigester 9:1→6:1 gereinigt wird. Man erhält 0,39 g kristallines (Z)-5-(2,5-Dimethoxy-3,4,6-trimethylphenyl)-3-methyl -2-penten-1-ol vom Schmelzpunkt 58,5-60° C. $^1$H-NMR ($CDCl_3$, δ): 1,22 (1H,OH), 1,65 (s,3H), 2,16 (s,6H), 2,25 (s,3H), 2,24 und 2,68 (je m,2H), 3,65 und 3,68 (je s,3H), 4,04 (t,2H), 5,46 (t,1H).

d3) Essigsäureanhydrid (0,2 ml) wird zusammen mit (Z)-5-(2,5-Dimethoxy-3,4,6-trimethylphenyl)-3-methyl -2-penten-1-ol (0,39 g; 1,4 mMol) in Pyridin (10 ml) bei 0° C gelöst. Eine Spatelspitze 4-Dimethylaminopyridin wird zugesetzt, und das Reaktionsgemisch wird 20 Minuten bei 0° C belassen. Danach wird auf 150 ml eiskalte 2N Salzsäure gegossen und mit Aether extrahiert. Die Extrakte werden mit Wasser gewaschen, kurz getrocknet und vom Lösungsmittel befreit. Es verbleiben 0,39 g (Z)-5-(2,5-Dimethoxy-3,4,6-trimethylphenyl)-3-methyl-2-pentenylacetat als fast farbloses Oel, das keiner weiteren Reinigung bedarf. IR: 2938, 1738, 1667, 1457, 1241 $cm^{-1}$.

e3) Eine Lösung von (Z)-5-(2,5-Dimethoxy-3,4,6-trimethylphenyl)-3-methyl -2-pentenylacetat (0,98 g) in Acetonitril (15 ml) wird, bei -20° C, tropfenweise mit einer Lösung von Cerammoniumnitrat (4,1 g) in Wasser (15 ml) behandelt. Nach 15 Minuten wird auf -10° C aufgewärmt und 1 Stunde bei dieser Temperatur belassen. Es wird mit Eiswasser verdünnt und zweimal mit Methylenchlorid extrahiert. Die Extrakte werden mit Wasser gewaschen, getrocknet und an Kieselgel mit Hexan/Essigester 95:5 chromatographiert. Es verbleiben 0,67 g eines hellgelben Oels. Das so erhaltene (Z)-3-Methyl-5-(2,4,5-trimethyl-3,6-dioxo-1,4 -cyclohexadienyl)-2-pentenylacetat zeigt im IR-Spektrum Banden bei 2944, 1738, 1642, 1234 $cm^{-1}$. MS: 290 ($M^+$), 248 ($-C_2H_2O$), 230 (-HOAc).

f3) Eine Lösung von 0,67 g (2,3 mMol) (Z)-3-Methyl-5-(2,4,5-trimethyl-3,6-dioxo-1,4 -cyclohexadienyl)-2-pentenylacetat in 20 ml THF wird tropfenweise mit 8,1 ml einer 0,5M wässrigen Natriumdithionit-Lösung versetzt. Nach beendeter Zugabe wird mit Wasser verdünnt und dreimal mit Chloroform extrahiert. Die vereinigten Extrakte werden mit Wasser gewaschen und vom Lösungsmittel befreit. Der Rückstand wird in Hexan trituriert und man erhält 0,57 g (Z)-5-(2,5-Dihydroxy-3,4,6-trimethylphenyl)-3-methyl -2-pentenylacetat als schwach gefärbtes Pulver vom Schmelzpunkt 116,5-117,5° C. $^1$H-NMR ($CDCl_3$, δ): 1,86 (s,3H), 2,07 (s,3H), 2,17, 2,18, 2,21 (je s,3H), 2,28 und 2,70 (je m,2H), 4,31 und 5,26 (je s,1H,OH), 4,58 (d,2H), 5,37 (t,1H).

E. Katalysatoren

## Beispiel 6

1) Eine Lösung von (R)-6,6′-Dimethyl-2,2′-bis(diphenylphosphino)-1,1′-biphenyl (0,22 g; 0,4 mMol) und π-Allylpalladiumchloriddimer (74 mg; 0,2 mMol) in Methanol (1,5 ml) wird nach 15 Minuten tropfenweise mit einer Lösung von Lithiumperchlorat (212 mg; 2 mMol) in Methanol (1 ml) behandelt. Die entstehende Suspension wird 12 Stunden bei Raumtemperatur weitergerührt. Danach wird mit Chloroform verdünnt, mit Wasser gewaschen und über Natriumsulfat getrocknet. Nach dem Filtrieren wird das Lösungsmittel am Rotationsverdampfer entfernt und der Rückstand aus Aceton umkristallisiert. Man erhält 280 mg farbloses [-[(R)-6,6′-Dimethyl-1,1′-biphenyl-2,2′-bis(diphenylphosphin)] -P,P′][(η-3)-2-propenyl]palladiumperchlorat mit einer optischen Drehung von +261,5° (DMSO, c = 0,4). $^1$H-NMR (DMSO, δ): 1,44, 1,50 (je s,3H), 2,94, 3,6, 4,01, 4,13 (je m,1H), 6,02 (tt,1H,meso-H), 6,7-7,7 (m,26H). 31P-NMR (DMSO, δ): 22,85 und 22,35 (je d,J(P-P)=49Hz).

2) Eine Lösung von (R)-6,6′-Dimethyl-2,2′-bis(diphenylphosphino)-1,1′-biphenyl (0,22 g; 0.4 mMol) und π-Allylpalladiumchloriddimer (74 mg; 0.2 mMol) in Methanol (1 ml) wird mit 680 mg (2 mMol) Natriumtetraphenylborat behandelt. Der entstehende Niederschlag wird nach 4 Stunden abgesaugt, mit 5 ml 50% wässrigem Methanol gewaschen und in 10 ml Aceton gelöst. Filtration der entstehenden Lösung durch Celit und anschliessendes Verdünnen mit 200 ml Aether ergibt einen praktisch farblosen Niederschlag (284 mg). Das so erhaltene [[(R)-6,6-Dimethyl-1,1′-biphenyl-2,2′-bis(diphenylphosphin)]-P,P′][(η-3)-2-propenyl]-palladiumtetraphenylborat weist im $^1$H-NMR Spektrum ($CDCl_3$) Banden (δ) bei 1,36 und 1,47 (je s,3H), 2,36,

3,50, 3,60 und 3,88 (je m,1H), 5,29 (tt;1H,meso-H), 6,75-7,60 (m,46H) ppm und im 31P-NMR (DMSO) Banden bei 22,35 und 22,80 (je d,J(P-P) = 51) ppm auf.

F. Cyclisierungen

Beispiel 7

Versuch 1

Eine Lösung von (E)-5-(2,5-Dihydroxy-3,4,6-trimethylphenyl)-3-methyl -2-pentenylacetat (125 mg; 0,43 mMol), (R)-6,6′-Dimethyl-2,2′-bis(diphenylphosphino-1,1′-biphenyl (7 mg, 3%) und π-Allylpalladiumchloriddimeres (2,3 mg, 1,5%) in Tetrahydrofuran (2 ml) wird 12 Stunden bei 65° C gehalten. Nach dem Abkühlen wird das Lösungsmittel entfernt und der Rückstand an 3 g Kieselgel mit Hexan/Essigester (99:1) gereinigt. Nach Kristallisation aus Pentan erhält man 81 mg (S)-3,4-Dihydro-2,5,7,8-tetramethyl-2-viny-l2H-[1]-benzopyran-6-ol in einer optischen Reinheit von 53%. IR: 3440, 1621, 1260 $cm^{-1}$. MS: 232 ($M^+$), 203 (-$C_2H_5$), 164 (-$CH_2=C(CH_3)CH=CH_2$), 136 (164-CO) und 121 (136-$CH_3$).

Versuch 2

Eine Lösung von 125 mg (0,43 mMol) (Z)-5-(2,5-Dihydroxy-3,4,6-trimethylphenyl)-3-methyl -2-pentenylacetat, 8 mg (3%) (S)-2,2′-Bis(diphenylphosphino)-1,1′-binaphthalin und 2,3 mg (1,5%) π-Allylpalladiumchloriddimeres in DMSO (3 ml) wird 17 Stunden bei 60° C belassen. Nach dem Erkalten wird mit Wasser verdünnt und mit Aether extrahiert. Die Extrakte werden mit Wasser gewaschen, getrocknet, eingeengt und der Rückstand wie in Versuch 1 gereinigt. Man erhält 86 mg (S)-3,4-Dihydro-2,5,7,8-tetramethyl-2-vinyl -2H-[1]-benzopyran-6-ol in einer optischen Reinheit von 36%.

Versuch 3

Eine Lösung von 92 mg (0,3 mMol) (E)-5-(3,6-Dihydroxy-2,4,5-trimethylphenyl) -2-pentenylmethylcarbonat, 5 mg (3%) (R)-6,6′-Dimethyl-2,2′-bis(diphenylphosphino)-1,1′-biphenyl und 1,7 mg (1,5%) π-Allylpalladiumchloriddimeres in 2 ml Toluol wird 20 Stunden auf 60° erwärmt. Nach dem Abkühlen wird das Lösungsmittel entfernt, und der Rückstand wie in Versuch 1 beschrieben gereinigt. Man erhält 54 mg (S)-3,4-Dihydro-2,5,7,8-tetramethyl-2-vinyl -2H-[1]benzopyran-6-ol in einer optischen Reinheit von 20%.

Versuch 4

Eine Lösung von 106 mg (0,3 mMol) (E)-5-(2,5-Dihydroxy-3,4,6-trimethylphenyl)-3-methyl -2-pentenylbenzoat, 5 mg (3%) (R)-6,6′-Dimethyl-2,2′-bis(diphenylphosphino)-1,1′-biphenyl und 1,7 mg (1,5%) π-Allylpalladiumchloriddimeres in 5 ml THF wird 24 Stunden auf 70° C erwärmt. Nach dem Abkühlen wird das Lösungsmittel entfernt und der Rückstand wie in Versuch 1 beschrieben gereinigt. Man erhält 39 mg (S)-3,4-Dihydro-2,5,7,8-tetramethyl-2-vinyl -2H-[1]-benzopyran-6-ol in einer optischen Reinheit von 56%.

Versuch 5

Eine Lösung von 106 mg (0,36 mMol) (E)-5-(2,5-Dihydroxy-3,4,6-trimethylphenyl)-3-methyl -2-pentenylacetat und 9 mg (3%) [[(R)-6,6′-Dimethyl-1,1′-biphenyl-2,2′-bis(diphenylphosphin)] -P,P′][(η-3)-2-propenyl]-palladiumperchlorat in 2 ml DMSO wird mit 15 mg Calciumhydrid versetzt und drei Tage bei Raumtemperatur gerührt. Danach wird in gesättigte Ammoniumchloridlösung gegossen und mit Aether extrahiert. Die vereinigten Extrakte werden mit Wasser gewaschen und getrocknet. Der nach Entfernen des Lösungsmittels verbleibende Rückstand wird wie in Versuch 1 beschrieben gereinigt. Man erhält 56 mg (S)-3,4-Dihydro-2,5,7,8-tetramethyl-2-vinyl -2H-[1]-benzopyran-6-ol in einer optischen Reinheit von 33%.

Versuch 6

Eine Lösung von 104 mg (0,36 mMol) (E)-5-(2,5-Dihydroxy-3,4,6-trimethylphenyl)-3-methyl -2-pentenylacetat, 11 mg (3%) [[(R)-6,6'-Dimethyl-1,1'-biphenyl-2,2'-bis(diphenylphosphin)] -P,P']-[(η-3)-2-propenyl]-palladiumtetraphenylborat und 100 mg 4-Dimethylaminopyridin in 2 ml DMSO wird 20 Stunden auf 60° erwärmt. Nach dem Abkühlen wird wie in Versuch 2 beschrieben aufgearbeitet und chromatographiert. Man erhält 63 mg (S)-3,4-Dihydro-2,5,7,8-tetramethyl-2-vinyl -2H-[1]-benzopyran-6-ol in einer optischen Reinheit von 53%.

## Ansprüche

1. Verfahren zur Herstellung von Vinylchromanen der Formel

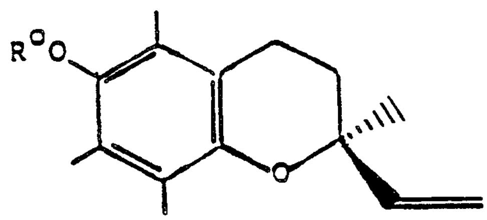

I

worin R° Wasserstoff oder eine abspaltbare Schutzgruppe darstellt, dadurch gekennzeichnet, dass man eine Verbindung der Formel

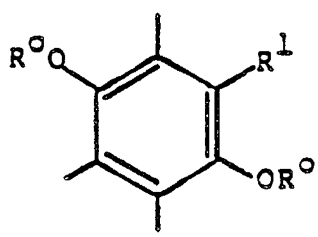

II

worin R° obige Bedeutung besitzt und $R^1$ den Rest $-CH_2CH_2-C(W)(CH_3)-CH=CH_2$ oder $-CH_2-CH_2-C(CH_3)=CH-CH_2W$ darstellt, worin W eine Abgangsgruppe ist,
mittels eines chiralen Uebergangsmetall-diphosphin-komplexes cyclisiert.

2. Verfahren nach Anspruch 1, dadurch gekennzeichnet, dass als Uebergangsmetall ein Metall der Gruppe 8 des periodischen Systems fungiert.

3. Verfahren nach Anspruch 2, dadurch gekennzeichnet. dass als Metall Rhodium, Palladium, Nickel oder Platin, insbesondere aber Rhodium oder Palladium fungiert.

4. Verfahren nach einem der Ansprüche 1-3, dadurch gekennzeichnet, dass man einen chiralen Rhodium-diphosphinkomplex der Formel
$[Rh(X)(Y)(L_{0,1,2})]_{1,2}$ III
worin bedeuten:
X: Halogenid, $BF_4^-$, $PF_6^-$, $ClO_4^-$, $NO_3^-$, $B(C_6H_5)_4^-$, oder ein Rest der Formel $Z-COO^-$, gegebenenfalls auf einem Träger fixiert

Z: 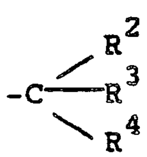 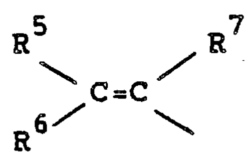 ; Aryl -

worin sind:
$R^2$, $R^3$, $R^4$: H; Halogen; nieder Alkyl; Aryl-nieder Alkyl; Perfluor-$C_{1-20}$-Alkyl; Aryl; $OR^8$; $-(CH_2)_n-COA$; AOC-$(CF_2)_n$, wenigstens einer von $R^2$, $R^3$ und $R^4$ ist $-OR^8$, Halogen oder Aryl.

$R^5$, $R^6$, $R^7$: H; Halogen; nieder Alkyl; Aryl-nieder Alkyl; Perfluor-$C_{1-20}$-Alkyl; Aryl; -$(CH_2)_n$-COA; AOC-$(CF_2)_n$.
$R^8$: H; nieder Alkyl; teilweise oder vollständig halogeniertes niederes Alkyl; Aryl; Aryl-nieder-Alkyl.
A: -OR'''; -NR''''$_2$
R''': H; nieder Alkyl; Aryl; Aryl-nieder Alkyl, Kation.
R'''': H; nieder Alkyl; Aryl; Aryl-nieder Alkyl.
n: Zahl 0 - 20,
Y: chiraler Diphosphinligand
L: neutraler Ligand.
verwendet.

5. Verfahren nach Anspruch 4, dadurch gekennzeichnet, dass man den chiralen Rhodium-diphosphinkomplex der Formel III mit X = Z-$COO^-$, Z = -$C(R^2R^3R^4)$ verwendet, worin Y und L obige Bedeutung haben.

6. Verfahren nach einem der Ansprüche 1-3, dadurch gekennzeichnet, dass ein Palladium-diphosphinkomplex der Formel
[Pd(X)($X^1$)(Y)] IV
worin X obige Bedeutung besitzt, $X^1$ ein $\pi$-allylischer Ligand vom Typ

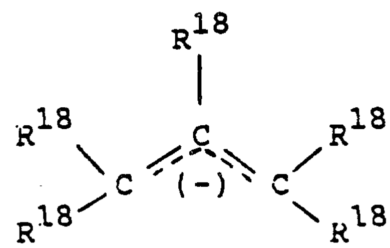

darstellt, worin $R^{18}$ Wasserstoff, Aryl, nieder Alkyl oder Benzyl bedeutet und Y obige Bedeutung hat, verwendet wird.

7. Verfahren nach Anspruch 4, 5 oder 6, dadurch gekennzeichnet, dass der chirale Diphosphinligand die Formel

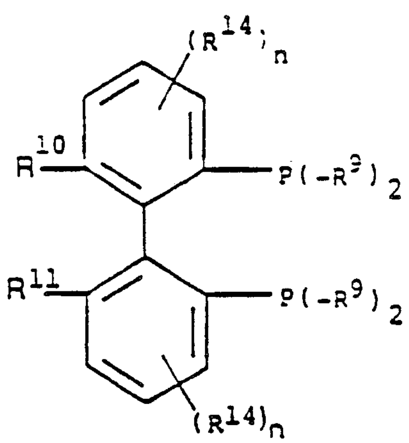

IX oder X

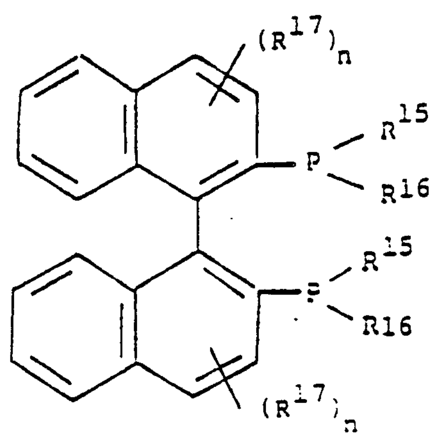

aufweist, worin:
$R^9$: Aryl, Cyclohexyl
$R^{10}$,$R^{11}$: H; nieder-Alkyl; nieder-Alkoxy; di-nieder-Alkylamino; geschütztes Hydroxymethyl. $R^{10}$ und $R^{11}$ können voneinander verschieden sein oder zusammen die folgenden Gruppen bedeuten:

$-(CH_2)_m$;  $-CH_2-O-CH_2-$;

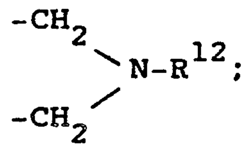

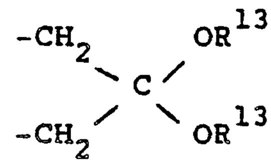

m: Zahl 3,4,5
$R^{12}$: nieder Alkyl, Aryl, Benzyl
$R^{13}$: nieder Alkyl; oder beide $R^{13}$ zusammen Di- oder Tri-methylen
$R^{14}$: $-CH_3$; nieder Alkoxy; di-nieder Alkyl-amino; Halogen
n: Zahl 0,1,2,3
$R^{15}$ $R^{16}$: Aryl, Cyclohexyl
$R^{17}$: Methyl, Aethyl, Halogen, -OH; $NH_2$, Acetylamino, Nitro, $-SO_3H$, bevorzugt in der 5,5′-Position.

8. Verfahren nach einem der Ansprüche 1-7, dadurch gekennzeichnet, dass man den Katalysator in situ herstellt.

9. Verfahren nach einem der Ansprüche 1-8, dadurch gekennzeichnet, dass $R^0$ Methyl, Acetyl oder gegebenenfalls substituiertes Benzyl darstellt.

10. Verfahren nach einem der Ansprüche 1-9, dadurch gekennzeichnet, dass durch W ein Ester, ein Halogenid oder eine Aryloxygruppe charakterisiert wird.

11. Verfahren nach einem der Ansprüche 1-10, dadurch gekennzeichnet. dass W einen Ester darstellt.

12. Verfahren nach einem der Ansprüche 1-11, dadurch gekennzeichnet, dass man bei Temperaturen von -20-100 °C, insbesondere bei 0° bis 60 °C, cyclisiert.

13. Verfahren nach einem der Ansprüche 1-12, dadurch gekennzeichnet, dass man in einem Lösungsmittel. insbesondere einem, gegebenenfalls halogenierten, aliphatischen oder aromatischen Kohlenwasserstoff, einem cyclischen Aether, einem Ester, einem Alkanol, Dimethylformamid, Dimethylsulfoxid oder Pyridin cyclisiert.

14. Verfahren nach einem der Ansprüche 1-13, dadurch gekennzeichnet, dass in Anwesenheit einer Base, insbesondere einer organischen Base cyclisiert wird.

15. Verfahren nach einem der Ansprüche 1-14, dadurch gekennzeichnet. dass man den Katalysator in einer Menge von ca. 0.01-10 Gew.%. vorzugsweise 1-5 Gew.% einsetzt.

16. Verfahren nach einem der Ansprüche 1-15, dadurch gekennzeichnet. dass man die erhaltene Verbindung der Formel I durch Hydroborierung und Oxidation in eine Verbindung der Formel

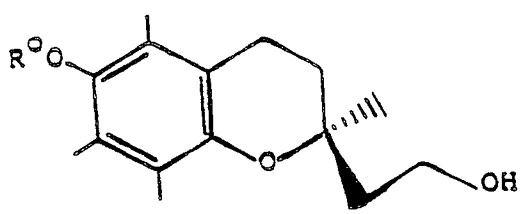

VI

worin $R^0$ Wasserstoff oder eine abspaltbare Schutzgruppe darstellt, überführt.

17. Verfahren nach einem der Ansprüche 1-16, dadurch gekennzeichnet, dass man die Verbindung der Formel VI in an sich bekannter Weise in (R,R,R)-α-Tocopherol überführt.

18. Verbindungen der Formel

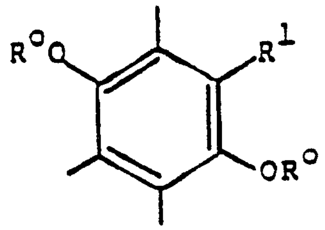

II

worin R° Wasserstoff oder eine abspaltbare Schutzgruppe und $R^1$ den Rest $-CH_2CH_2-C(W)(CH_3)-CH=CH_2$ oder $-CH_2-CH_2-C(CH_3)=CH-CH_2-W$ darstellen.

19. Verbindungen der Formel II gemäss Anspruch 18, worin W eine Estergruppierung ist.

20. Verbindungen nach Anspruch 19, dadurch gekennzeichnet, dass W ein Alkanoat, Methylcarbonat oder Carbonat ist.